# EUROPEAN PATENT SPECIFICATION

(11) **EP 3 018 478 B1**
(45) Date of publication and mention of the grant of the patent: **19.08.2020**
(21) Application number: 14003703.7
(22) Date of filing: 04.11.2014
(51) Int. Cl.: G01N 33/564, G01N 33/68

(54) **A NOVEL DIAGNOSTICALLY RELEVANT AUTOANTIBODY**
NEUARTIGER, DIAGNOSTISCH RELEVANTER AUTOANTIKÖRPER
NOUVEL AUTOANTICORPS D'INTÉRÊT POUR LES DIAGNOSTICS

(43) Date of publication of application: 11.05.2016
(73) Proprietor: Euroimmun Medizinische Labordiagnostika AG, 23560 Lübeck (DE)
(72) Inventor: Stöcker, Winfried, 23627 Groß Grönau (DE); Komorowski, Lars, 23909 Ratzeburg (DE); Scharf, Madeleine, 23564 Lübeck (DE); Miske, Ramona, 23552 Lübeck (DE); Denno, Yvonne, 23558 Lübeck (DE); Dettmann, Inga-Madeleine, 23623 Ahrensbök (DE); Probst, Christian, 23909 Ratzeburg (DE); Tiede, Stephan, 21923 Lüdersdorf (DE)

(56) References cited:
- EP-A1- 2 952 898
- WO-A1-2005/017151
- US-A1- 2010 035 360
- DATABASE MEDLINE [Online] US NATIONAL LIBRARY OF MEDICINE (NLM), BETHESDA, MD, US; 2009, KURIEN BIJI T: "Affinity purification of autoantibodies from an antigen strip excised from a nitrocellulose protein blot.", XP002737576, Database accession no. NLM19378059 & METHODS IN MOLECULAR BIOLOGY (CLIFTON, N.J.) 2009, vol. 536, 2009, pages 201-211, ISSN: 1064-3745
- SVEN JARIUS ET AL: "Antibodies to the inositol 1,4,5-trisphosphate receptor type 1 (ITPR1) in cerebellar ataxia", JOURNAL OF NEUROINFLAMMATION, BIOMED CENTRAL LTD., LONDON, GB, vol. 11, no. 1, 11 December 2014 (2014-12-11), page 206, XP021210086, ISSN: 1742-2094, DOI: 10.1186/S12974-014-0206-3
- Giulia Berzero ET AL: "Clinical/Scientific Notes", , 3 February 2017 (2017-02-03), XP55471854, Retrieved from the Internet: URL:https://www.ncbi.nlm.nih.gov/pmc/artic les/PMC5292928/pdf/NEURIMMINFL2016011544.p df [retrieved on 2018-05-02]

### SEQUENCE LISTING

<110> EUROIMMUN
<120> A novel diagnostically relevant autoantibody
<130> P00037
<160> 3
<170> BiSSAP 1.3
<210> 1
   <211> 16190
   <212> DNA
   <213> Artificial Sequence
<220>
   <223> Expression vector
<400> 1
   aggccaccat gtctgacaaa atgtcgagtt tcctacatat tggagacatt tgttctctgt      60
   atgcggaggg atctacgaat ggatttatca gcaccttagg cttggttgat gaccgttgtg     120
   ttgtacagcc agaagccggg gaccttaaca atccacccaa gaaattcaga gactgcctct     180
   ttaagctatg tcctatgaat cgatactccg cacagaaaca gttctggaaa gctgctaagc     240
   ccggggccaa cagcactaca gatgcagtgc tgctcaacaa attgcatcat gctgcagact     300
   tggaaaagaa gcagaatgag acagaaaaca ggaaattgtt ggggaccgtc atccaatatg     360
   gcaacgtgat ccagctcctg catttgaaaa gcaataaata cctgactgtg aataagaggc     420
   tcccagcctt gctagagaag aacgccatga gggtgacgtt ggacgaggct ggaaatgaag     480
   ggtcctggtt ttacattcaa ccattctaca agcttcgctc catcggagac agtgtggtca     540
   taggcgacaa ggtagttttg aatcctgtca atgctggcca gcctctaaat gccagcagtc     600
   atcagctggt ggataaccca ggctgcaatg aggtcaactc cgtcaactgt aatacaagct     660
   ggaagatatt gcttttcatg aaatggagtg ataacaaaga cgatattctc aaaggaggtg     720
   atgtggtgag gctcttccat gccgagcaag agaagtttct cacctgcgat gagcaccgga     780
   agaagcagca tgtgttcctg aggaccaccg gcaggcagtc agccacatcg gccaccagtt     840
   ccaaagccct gtgggaagtg gaggtagtcc agcacgaccc atgtcggggt ggagctgggt     900
   actggaatag cctcttccgg ttcaagcacc tggctacagg gcattacttg gctgcagagg     960
   tagaccctga ctttgaggaa gaatgcctgg agtttcagcc ctcagtggac cctgatcagg    1020
   atgcatctcg gagtaggttg agaaacgcgc aagaaaaaat ggtatactct ctggtctccg    1080
   tgcctgaagg caacgacatc tcctccatct ttgagctaga ccccacgact ctgcgtggag    1140
   gtgacagcct tgtcccaagg aactcctatg tccgtctcag acacctgtgc accaacacct    1200
   gggtacacag cacaaacatc cccatctaca aggaagagga gaagcctgtg atgctgaaaa    1260
   ttggtacctc tcccctgaag gaggacaagg aagcatttgc catagttcct gtttcccctg 1320
   ctgaggttcg ggacctggac tttgccaatg atgccagcaa ggtgctgggc tccatcgctg 1380
   ggaagttgga aaagggcaca atcacccaga atgagagaag gtctgtcacg aagcttttgg 1440
   aagacttggt ttactttgtc acgggtggaa ctaactctgg ccaagacgtg cttgaagttg 1500
   tcttctctaa gcccaatcga gagcggcaga agctgatgag ggaacagaat attctcaagc 1560
   agatcttcaa gctgttgcag gcccccttca cggactgcgg ggatggcccg atgcttcggc 1620
   tggaggagct gggggatcag cgccatgctc ctttcagaca tatttgccga ctctgctaca 1680
   gggtcctgcg acactcacag caagactaca ggaagaacca ggagtacata gccaagcagt 1740
   ttggcttcat gcagaagcag attggctatg acgtgctggc cgaagacacc atcactgccc 1800
   tgctccacaa caaccggaaa ctcctggaga agcacatcac cgcggcagag attgacacgt 1860
   ttgtcagcct ggtgcgaaag aacagggagc ccaggttctt ggattacctc tctgacctct 1920
   gcgtatccat gaacaagtca atccctgtga cacaggagct catctgtaaa gctgtgctca 1980
   atcccaccaa tgctgacatc ctgattgaga ccaagctggt tctttctcgt tttgagtttg 2040
   aaggcgtttc cactggagag aatgctctgg aagccgggga ggatgaggaa gaggtgtggc 2100
   tgttctggag ggacagcaac aaagagatcc gtagtaagag tgtccgggaa ttggcgcaag 2160
   atgctaaaga gggacagaag gaagacaggg acatcctcag ctactacaga tatcagctga 2220
   acctctttgc aaggatgtgt ctggaccgcc agtacctggc catcaatgaa atctccgggc 2280
   agctggatgt tgatctcatt ctccgctgca tgtctgacga gaacctcccc tacgacctca 2340
   gggcatcctt ttgccgcctc atgcttcaca tgcatgtgga ccgagatccc caagagcagg 2400
   tgacacctgt gaaatatgcc cgactgtggt cagaaattcc ctctgagatc gccattgatg 2460
   actatgacag cagtggaaca tccaaagatg aaattaagga gaggtttgca cagacgatgg 2520
   agtttgtgga ggagtaccta agagatgtgg tttgtcaaag attccccttc tctgataagg 2580
   agaaaaataa gctcacgttt gaggttgtga acttagccag gaatctcata tactttggtt 2640
   tctacaactt ttctgacctt ctccgattaa ccaagatcct cttggcaatc ttagactgtg 2700
   tccatgtgac cactatcttc cccattagca agatgacaaa aggagaagag aataaaggca 2760
   gtaacgtgat gaggtctatc catggcgttg gggagctgat gacccaggtg gtgctgcggg 2820
   gaggaggctt cttgcccatg actcccatgg ctgcggcccc tgaaggaaat gtgaagcagg 2880
   cagagccaga gaaagaggac atcatggtca tggacaccaa gttgaagatc attgaaatac 2940
   tccagtttat tttgaatgtg agattggatt ataggatctc ctgcctcctg tgtatattta 3000
   agcgagagtt tgatgaaagc aattcccagt catcagaaac atcctccgga aacagcagcc 3060
   aggaagggcc aagtaatgtg ccaggtgctc ttgactttga acacattgaa gaacaagcgg 3120
   aaggcatctt tggaggaagt gaggagaaca cacctttgga cctggatgac catggtggca 3180
   gaaccttcct cagggtcctg ctccacttga caatgcatga ctacccaccc ctggtgtctg 3240
   gggccctgca gctcctcttt cggcacttca gccagaggca ggaggtcctt caggccttca 3300
   aacaggttca actgctggtt actagccaag atgtggacaa ctacaaacag atcaagcaag 3360
   acttggacca actaaggtcc attgtggaga agtctgagct ctgggtgtac aaaggccaag 3420
   gtcccgatga gcctatggac ggagcctccg gtgaaaatga gcataagaaa accgaggagg 3480
   ggacgagcaa gccactgaag cacgagagca ccagcagcta caactaccga gtggtgaaag 3540
   agattttgat tcgacttagc aagctctgcg tgcaggagag cgcgtcggtg aggaagagcc 3600
   ggaagcagca gcaacgactg ctgaggaaca tgggcgcaca cgctgtggtg ctggagctgc 3660
   tgcagatccc ctacgagaag gccgaagaca caaagatgca agagatcatg cggctggctc 3720
   atgaattttt gcagaatttc tgtgcaggca accagcagaa tcaagctttg ctgcataaac 3780
   acataaacct gtttctcaac ccagggattc tggaggcagt gacgatgcag cacatcttca 3840
   tgaacaactt ccagctgtgc agtgagatca acgagagagt ggtccagcac tttgttcact 3900
   gcatagagac ccacggtcga aacgtccagt atatcaagtt tctccagacg attgtcaagg 3960
   cagaagggaa attcattaaa aagtgccaag acatggtcat ggctgagctt gtcaactctg 4020
   gagaggacgt cctcgtgttc tacaatgaca gagcctcttt ccagactctg atccagatga 4080
   tgcggtccga gcgtgaccgg atggatgaga acagccctct catgtaccac atccatctgg 4140
   tggagctctt ggccgtgtgc acagagggca agaatgtgta cacggagatc aagtgcaact 4200
   ccttgctccc gctcgatgac atcgttcgtg tggtcactca tgaagactgc atccccgagg 4260
   ttaagatcgc ttacattaac ttcctgaatc actgctatgt ggatacggag gtggagatga 4320
   aggagattta cacaagcaac cacatgtgga agttgtttga gaatttcctc gtggacatct 4380
   gcagggcctg taacaacaca agcgacagga agcacgcaga ctccattctg gagaagtacg 4440
   tcactgaaat cgtgatgagc atcgtcacca ccttcttcag ctctcccttc tcagaccaga 4500
   gcaccactct gcagacccgc cagcctgtct ttgtgcaact cctgcaaggc gtgttccgag 4560
   tttaccactg caactggctg atgccgagcc aaaaagcctc ggtggagagc tgcatccggg 4620
   tgctctctga cgtagccaag agccgggcca tagccattcc tgttgacctg gacagccaag 4680
   tcaacaacct cttcctgaag tcccacaaca ttgtgcagaa aacagccctg aactggcggt 4740
   tatcagcccg aaacgccgct cgcagagact ctgtactggc agcatccaga gactaccgaa 4800
   atatcattga gaggttacag gacatcgtgt ctgccctaga ggaccggctc aggcccctgg 4860
   tgcaggctga gctgtctgtg ctcgtggatg ttctacacag accagaactg ctcttccccg 4920
   agaacacgga tgccaggagg aaatgtgaga gtggaggttt catctgcaag ctaataaaac 4980
   ataccaagca actgctggag gagaatgaag agaaactatg cattaaagtc ttacagaccc 5040
   tcagggaaat gatgaccaaa gacagaggct atggagagaa gcaaatttcc attgatgaat 5100
   cggaaaatgc cgagctgcca caggcaccgg aagctgagaa ctccacagag caggagcttg 5160
   aaccaagtcc acccctgagg caactggaag accataaaag gggtgaggca ctccgacaaa 5220
   ttttggtcaa ccgttactat ggaaacatca gaccttcagg aagaagagag agccttacca 5280
   gctttggcaa tggcccacta tcaccaggag gacccagcaa gcctggtgga ggagggggag 5340
   gtcctggatc tagttccaca agcaggggtg agatgagcct ggctgaggtt cagtgtcacc 5400
   tcgacaagga gggggcctcc aacctggtca tcgatctcat aatgaatgca tccagtgacc 5460
   gagtattcca tgaaagcatt ctgctggcca tcgcacttct ggaaggaggc aacaccacca 5520
   tccagcactc gtttttctgc cggctgacag aagataagaa atcagagaag ttcttcaagg 5580
   ttttttacga tcgaatgaag gtggcccagc aggaaatcaa ggcgacagtg acagtgaaca 5640
   ccagcgactt gggaaacaaa aagaaagatg atgaagtgga cagggatgcc ccgtcccgga 5700
   agaaagccaa agagcccaca acacagataa cagaagaggt ccgggatcag ctcctggaag 5760
   catctgctgc caccaggaaa gcctttacca ccttccggag ggaggccgac cctgatgacc 5820
   attaccagtc tggggagggc acccaggcta caaccgacaa agccaaggat gacctagaga 5880
   tgagcgctgt catcaccatc atgcagccta tcctgcgctt cctgcagctg ctgtgtgaaa 5940
   accacaaccg agatctgcag aatttccttc gttgccaaaa taataagacc aactacaatt 6000
   tggtgtgtga gacactgcag tttctggact gtatttgtgg gagcacaacc ggaggccttg 6060
   gtcttcttgg actgtacata aatgaaaaga atgtagcact tatcaaccaa accctggaga 6120
   gtctgacgga gtactgtcaa gggccttgcc atgagaacca gaactgcatc gccacccacg 6180
   agtccaatgg catcgatatc atcacagccc tcatcctcaa tgatatcaac cctctgggaa 6240
   agaagcggat ggacctggtg ttagaactga agaacaatgc ttcgaagctg ctactggcca 6300
   tcatggaaag cagacacgat agtgaaaatg cagagaggat cctgtacaac atgaggccca 6360
   aggagctggt ggaagtgatc aagaaggcct acatgcaagg tgaagtggaa tttgaggatg 6420
   gggagaacgg tgaggatgga gctgcctcac ccaggaacgt gggccacaac atctacatcc 6480
   tcgctcacca gttggctcgg cataacaaag aacttcaaac catgctgaaa cctggaggcc 6540
   aggtggatgg ggatgaagct ctggagttct acgcgaagca cacagcacaa attgagattg 6600
   tcagactgga ccggacaatg gaacagatcg tcttccctgt gcccagcatc tgtgaattcc 6660
   tgactaagga atcgaaactt cgaatatatt acaccacaga gcgggatgag caaggtagca 6720
   agatcaatga cttcttcctg cgctccgagg acctctttaa cgagatgaac tggcagaaga 6780
   aacttcgagc ccagcctgtc ttgtactggt gtgcccgaaa catgtctttc tggagcagca 6840
   tctccttcaa cctggccgtc ctgatgaacc tgctggtggc gtttttctat ccatttaaag 6900
   gagtgagggg aggaacacta gagccacact ggtcaggcct cctgcggaca gccatgctca 6960
   tctctctggc cattgtcatt gctctgccca agccccacgg catccgggcc ttaattgctt 7020
   ctacaatcct acgactgata ttttcagttg ggttgcagcc cacactgttt ctgctgggag 7080
   ctttcaatgt ctgcaataaa atcatcttcc tgatgagctt tgtgggcaac tgtgggacct 7140
   tcaccagagg ctaccgggcc atggttctgg atgtggagtt cctctatcat ttgctgtatc 7200
   tactcatctg tgccatgggc ctcttcgtac atgagttctt ctatagcttg ctgctttttg 7260
   atttagtgta cagagaggag actttgctta atgtcattaa aagtgtcacc cgcaatggac 7320
   ggtccatcat cttgacagcg gtcctggctc tgatcctggt ttacctgttc tcaattgtgg 7380
   gctatctgtt cttcaaggat gactttatct tggaagtaga taggttgccc aatgaaacag 7440
   ctgttccaga aactggcgag agtttggcca acgatttcct gtactctgat gtgtgcaggg 7500
   tagagacggg ggagaactgc acctctcctg cacccaaaga agagctgctc cctgccgaag 7560
   aaacggaaca ggataaggaa cacacgtgtg agaccctgct catgtgcatc gtcactgttc 7620
   tgagtcacgg gctgcggagt gggggagggg taggagacgt gctcaggaag ccatccaaag 7680
   aggagcctct gtttgctgca agggtgatct acgacctcct cttcttcttc atggtcatca 7740
   tcatcgtcct gaacctgatt ttcggggtca tcatcgacac ctttgctgac ctgaggagtg 7800
   agaagcaaaa gaaggaggag atcttaaaaa ccacgtgctt catctgcggc ttggaaaggg 7860
   acaagtttga caataagact gtcacctttg aagagcacat caaggaagaa cacaacatgt 7920
   ggcactatct gtgcttcatc gtgctggtga aagtgaagga ctccacagag tacaccgggc 7980
   ctgagagtta cgtggcagag atgatcaggg aaagaaacct tgattggttc cccagaatga 8040
   gagccatgtc cctggtcagc agcgattctg aaggggaaca gaacgagctg aggaacctgc 8100
   aggagaagct ggagtctacc atgaagctgg tcaccaatct ttctggccag ctgtcagaac 8160
   taaaggacca gatgacagaa cagaggaagc agaaacaaag aatcggcctt ctaggacatc 8220
   ctcctcacat gaatgtcaac ccacagcagc cggcctaggc ctcatgggcc ggcgcgccca 8280
   ccaccaccac caccactgac acgcaaggcc ggatccagac atgataagat acattgatga 8340
   gtttggacaa accacaacta gaatgcagtg aaaaaaatgc tttatttgtg aaatttgtga 8400
   tgctattgct ttatttgtaa ccattataag ctgcaataaa caagttaaca acaacaattg 8460
   cattcatttt atgtttcagg ttcaggggga ggtgtgggag gttttttaaa gcaagtaaaa 8520
   cctctacaaa tgtggtatgg ctgattatga tccggctgcc tcgcgcgttt cggtgatgac 8580
   ggaccggtcc agatatacgc gttgacattg attattgact agttattaat agtaatcaat 8640
   tacggggtca ttagttcata gcccatatat ggagttccgc gttacataac ttacggtaaa 8700
   tggcccgcct ggctgaccgc ccaacgaccc ccgcccattg acgtcaataa tgacgtatgt 8760
   tcccatagta acgccaatag ggactttcca ttgacgtcaa tgggtggagt atttacggta 8820
   aactgcccac ttggcagtac atcaagtgta tcatatgcca agtacgcccc ctattgacgt 8880
   caatgacggt aaatggcccg cctggcatta tgcccagtac atgaccttat gggactttcc 8940
   tacttggcag tacatctacg tattagtcat cgctattacc atggtgatgc ggttttggca 9000
   gtacatcaat gggcgtggat agcggtttga ctcacgggga tttccaagtc tccaccccat 9060
   tgacgtcaat gggagtttgt tttggcacca aaatcaacgg gactttctaa aatgtcgtaa 9120
   caactccgcc ccattgacgc aaatgggcgg taggcgtgta cggtgggagg tctatataag 9180
   cagagctctc tggctaacta gagaacccac tgcttactgg cttatcgaaa ttaatacgac 9240
   tcactatagg gagacccaag ctggctagcg tttaaactta agctagcttg gtacccgggg 9300
   atcctctagg gcctctgagc tattccagaa gtagtgaaga ggcttttttg gaggcctagg 9360
   cttttgcaaa aagctccgga tcgatcctga gaacttcagg gtgagtttgg ggacccttga 9420
   ttgttctttc tttttcgcta ttgtaaaatt catgttatat ggagggggca aagttttcag 9480
   ggtgttgttt agaatgggaa gatgtccctt gtatcaccat ggaccctcat gataattttg 9540
   tttctttcac tttctactct gttgacaacc attgtctcct cttattttct tttcattttc 9600
   tgtaactttt tcgttaaact ttagcctgca tttgtaacga atttttaaat tcacttttgt 9660
   ttatttgtca gattgtaagt actttctcta atcacttttt tttcaaggca atcagggtat 9720
   attatattgt acttcagcac agttttagag aacaattgtt ataattaaat gataaggtag 9780
   aatatttctg catataaatt ctggctggcg tggaaatatt cttattggta gaaacaacta 9840
   catcctggtc atcatcctgc ctttctcttt atggttacaa tgatatacac tgtttgagat 9900
   gaggataaaa tactctgagt ccaaaccggg cccctctgct aaccatgttc atgccttctt 9960
   ctttttccta cagctcctgg gcaacgtgct ggttattgtg ctgtctcatc attttggcaa 10020
   agaattgtaa tacgactcac tatagggcga attgatatgt ctagattaga taaaagtaaa 10080
   gtgattaaca gcgcattaga gctgcttaat gaggtcggaa tcgaaggttt aacaacccgt 10140
   aaactcgccc agaagctcgg tgtagagcag cctacattgt attggcatgt aaaaaataag 10200
   cgggctttgc tcgacgcctt agccattgag atgttagata ggcaccatac tcacttttgc 10260
   cctttagaag gggaaagctg gcaagatttt ttacgtaata acgctaaaag ttttagatgt 10320
   gctttactaa gtcatcgcga tggagcaaaa gtacatttag gtacacggcc tacagaaaaa 10380
   cagtatgaaa ctctcgaaaa tcaattagcc tttttatgcc aacaaggttt ttcactagag 10440
   aatgcattat atgcactcag cgctgtgggg cattttactt taggttgcgt attggaagat 10500
   caagagcatc aagtcgctaa agaagaaagg gaaacaccta ctactgatag tatgccgcca 10560
   ttattacgac aagctatcga attatttgat caccaaggtg cagagccagc cttcttattc 10620
   ggccttgaat tgatcatatg cggattagaa aaacaactta aatgtgaaag tgggtccgcg 10680
   tacagcggat cccgggaatt cagatcttat taaagcagaa cttgtttatt gcagcttata 10740
   atggttacaa ataaagcaat agcatcacaa atttcacaaa taaagcattt ttttcactgc 10800
   attctagttg tggtttgtcc aaactcatca atgtatctta tcatgtctgg tcgaatccct 10860
   taagcacttt tcggggaaat gtgcgcggaa cccctatttg tttatttttc taaatacatt 10920
   caaatatgta tccgctcatg agacaataac cctgataaat gcttcaataa tattgaaaaa 10980
   ggaagagtat gagtattcaa catttccgtg tcgcccttat tccctttttt gcggcatttt 11040
   gccttcctgt ttttgctcac ccagaaacgc tggtgaaagt aaaagatgct gaagatcagt 11100
   tgggtgcacg agtgggttac atcgaactgg atctcaacag cggtaagatc cttgagagtt 11160
   ttcgccccga agaacgtttt ccaatgatga gcacttttaa agttctgcta tgtggcgcgg 11220
   tattatcccg tgttgacgcc gggcaagagc aactcggtcg ccgcatacac tattctcaga 11280
   atgacttggt tgagtactca ccagtcacag aaaagcatct tacggatggc atgacagtaa 11340
   gagaattatg cagtgctgcc ataaccatga gtgataacac tgcggccaac ttacttctga 11400
   caacgatcgg aggaccgaag gagctaaccg cttttttgca caacatgggg gatcatgtaa 11460
   ctcgccttga tcgttgggaa ccggagctga atgaagccat accaaacgac gagcgtgaca 11520
   ccacgatgcc tgcagcaatg gcaacaacgt tgcgcaaact attaactggc gaactactta 11580
   ctctagcttc ccggcaacaa ttaatagact ggatggaggc ggataaagtt gcaggaccac 11640
   ttctgcgctc ggcccttccg gctggctggt ttattgctga taaatctgga gccggtgagc 11700
   gtgggtctcg cggtatcatt gcagcactgg ggccagatgg taagccctcc cgtatcgtag 11760
   ttatctacac gacggggagt caggcaacta tggatgaacg aaatagacag atcgctgaga 11820
   taggtgcctc actgattaag cattggtaac tgtcagacca agtttactca tatatacttt 11880
   agattgattt aaaacttcat ttttaattta aaaggatcta ggtgaagatc ctttttgata 11940
   atctcatgac caaaatccct taacgtgagt tttcgttcca ctgagcgtca gaccccgtag 12000
   aaaagatcaa aggatcttct tgagatcctt tttttctgcg cgtaatctgc tgcttgcaaa 12060
   caaaaaaacc accgctacca gcggtggttt gtttgccgga tcaagagcta ccaactcttt 12120
   ttccgaaggt aactggcttc agcagagcgc agataccaaa tactgtcctt ctagtgtagc 12180
   cgtagttagg ccaccacttc aagaactctg tagcaccgcc tacatacctc gctctgctaa 12240
   tcctgttacc agtggctgct gccagtggcg ataagtcgtg tcttaccggg ttggactcaa 12300
   gacgatagtt accggataag gcgcagcggt cgggctgaac ggggggttcg tgcacacagc 12360
   ccagcttgga gcgaacgacc tacaccgaac tgagatacct acagcgtgag ctatgagaaa 12420
   gcgccacgct tcccgaaggg agaaaggcgg acaggtatcc ggtaagcggc agggtcggaa 12480
   caggagagcg cacgagggag cttccagggg gaaacgcctg gtatctttat agtcctgtcg 12540
   ggtttcgcca cctctgactt gagcgtcgat ttttgtgatg ctcgtcaggg gggcggagcc 12600
   tatggaaaaa cgccagcaac gcggcctttt tacggttcct ggccttttgc tggccttgaa 12660
   gctgtccctg atggtcgtca tctacctgcc tggacagcat ggcctgcaac gcgggcatcc 12720
   cgatgccgcc ggaagcgaga agaatcataa tggggaaggc catccagcct cgcgtcgcga 12780
   acgccagcaa gacgtagccc agcgcgtcgg ccccgagatg cgccgcgtgc ggctgctgga 12840
   gatggcggac gcgatggata tgttctgcca agggttggtt tgcgcattca cagttctccg 12900
   caagaattga ttggctccaa ttcttggagt ggtgaatccg ttagcgaggt gccgccctgc 12960
   ttcatccccg tggcccgttg ctcgcgtttg ctggcggtgt ccccggaaga aatatatttg 13020
   catgtcttta gttctatgat gacacaaacc ccgcccagcg tcttgtcatt ggcgaattcg 13080
   aacacgcaga tgcagtcggg gcggcgcggt ccgaggtcca cttcgcatat taaggtgacg 13140
   cgtgtggcct cgaacaccga gcgaccctgc agcgacccgc ttaacagcgt caacagcgtg 13200
   ccgcagatcc cggggggcaa tgagatatga aaaagcctga actcaccgcg acgtctgtcg 13260
   agaagtttct gatcgaaaag ttcgacagcg tctccgacct gatgcagctc tcggagggcg 13320
   aagaatctcg tgctttcagc ttcgatgtag gagggcgtgg atatgtcctg cgggtaaata 13380
   gctgcgccga tggtttctac aaagatcgtt atgtttatcg gcactttgca tcggccgcgc 13440
   tcccgattcc ggaagtgctt gacattgggg aattcagcga gagcctgacc tattgcatct 13500
   cccgccgtgc acagggtgtc acgttgcaag acctgcctga aaccgaactg cccgctgttc 13560
   tgcagccggt cgcggaggcc atggatgcga tcgctgcggc cgatcttagc cagacgagcg 13620
   ggttcggccc attcggaccg caaggaatcg gtcaatacac tacatggcgt gatttcatat 13680
   gcgcgattgc tgatccccat gtgtatcact ggcaaactgt gatggacgac accgtcagtg 13740
   cgtccgtcgc gcaggctctc gatgagctga tgctttgggc cgaggactgc cccgaagtcc 13800
   ggcacctcgt gcacgcggat ttcggctcca acaatgtcct gacggacaat ggccgcataa 13860
   cagcggtcat tgactggagc gaggcgatgt tcggggattc ccaatacgag gtcgccaaca 13920
   tcttcttctg gaggccgtgg ttggcttgta tggagcagca gacgcgctac ttcgagcgga 13980
   ggcatccgga gcttgcagga tcgccgcggc tccgggcgta tatgctccgc attggtcttg 14040
   accaactcta tcagagcttg gttgacggca atttcgatga tgcagcttgg gcgcagggtc 14100
   gatgcgacgc aatcgtccga tccggagccg ggactgtcgg gcgtacacaa atcgcccgca 14160
   gaagcgcggc cgtctggacc gatggctgtg tagaagtact cgccgatagt ggaaaccgac 14220
   gccccagcac tcgtccggat cgggagatgg gggaggctaa ctgaaacacg gaaggagaca 14280
   ataccggaag gaacccgcgc tatgacggca ataaaaagac agaataaaac gcacgggtgt 14340
   tgggtcgttt gttcataaac gcggggttcg gtcccagggc tggcactctg tcgatacccc 14400
   accgagaccc cattggggcc aatacgcccg cgtttcttcc ttttccccac cccacccccc 14460
   aagttcgggt gaaggcccag ggctcgcagc caacgtcggg gcggcaggcc ctgccatagc 14520
   cactggcccc gtgggttagg gacggggtcc cccatgggga atggtttatg gttcgtgggg    14580
   gttattattt tgggcgttgc gtggggtcag gtccacgact ggactgagca gacagaccca    14640
   tggtttttgg atggcctggg catggaccgc atgtactggc gcgacacgaa caccgggcgt    14700
   ctgtggctgc caaacacccc cgacccccaa aaaccaccgc gcggatttct ggcgtgccaa    14760
   gctagtcgac gatatcgtac gctcttcgct cagcacgtga cctggtgcat gcgtatactg    14820
   cttcgaccaa ttctcatgtt tgacagctta tcatcgcaga tccgggcaac gttgttgcca    14880
   ttgctgcagg cgcagaactg gtaggtatgg aagatctata cattgaatca atattggcaa    14940
   ttagccatat tagtcattgg ttatatagca taaatcaata ttggctattg gccattgcat    15000
   acgttgtatc tatatcataa tatgtacatt tatattggct catgtccaat atgaccgcca    15060
   tgttgacatt gattattgac tagttattaa tagtaatcaa ttacggggtc attagttcat    15120
   agcccatata tggagttccg cgttacataa cttacggtaa atggcccgcc tggctgaccg    15180
   cccaacgacc cccgcccatt gacgtcaata atgacgtatg ttcccatagt aacgccaata    15240
   gggactttcc attgacgtca atgggtggag tatttacggt aaactgccca cttggcagta    15300
   catcaagtgt atcatatgcc aagtccgccc cctattgacg tcaatgacgg taaatggccc    15360
   gcctggcatt atgcccagta catgacctta cgggactttc ctacttggca gtacatctac    15420
   gtattagtca tcgctattac catggtgatg cggttttggc agtacaccaa tgggcgtgga    15480
   tagcggtttg actcacgggg atttccaagt ctccacccca ttgacgtcaa tgggagtttg    15540
   ttttggcacc aaaatcaacg ggactttcca aaatgtcgta ataaccccgc cccgttgacg    15600
   caaatgggcg gtaggcgtgt acggtgggag gtctatataa gcagagctct ccctatcagt    15660
   gatagagatc tccctatcag tgatagagag cttgcgttta gtgaaccgtc agatctctag    15720
   aagctgggta ccagctgcta gcaagcttgg aggcgctgcg acgctgcctt cgccccgtgc    15780
   cccgctccgc cgccgcctcg cgccgcccgc cccggctctg actgaccgcg ttactcccac    15840
   aggtgagcgg gcgggacggc ccttctcctt cgggctgtaa ttagcgcttg gtttaatgac    15900
   ggcttgtttc ttttctgtgg ctgcgtgaaa gccttgaggg gctccgggag ggccctttgt    15960
   gcggggggag cggctcgggg ctgtccgcgg ggggacggct gccttcgggg gggacggggc    16020
   agggcggggt tcggcttctg gcgtgtgacc ggcggctcta gagcctctgc taaccatgtt    16080
   catgccttct tctttttcct acagctcctg ggcaacgtgc tggttattgt gctgtctcat    16140
   cattttggca aagaattgga tcggaccgaa ataggagata taggccgtca               16190
<210> 2
   <211> 1251
   <212> PRT
   <213> Artificial Sequence
<220>
   <223> ITPR1 fragment 1-1251
<400> 2
   Met Ser Asp Lys Met Ser Ser Phe Leu His Ile Gly Asp Ile Cys Ser
   1               5                   10                  15
   Leu Tyr Ala Glu Gly Ser Thr Asn Gly Phe Ile Ser Thr Leu Gly Leu
               20                  25                  30
   Val Asp Asp Arg Cys Val Val Gln Pro Glu Thr Gly Asp Leu Asn Asn
           35                  40                  45
   Pro Pro Lys Lys Phe Arg Asp Cys Leu Phe Lys Leu Cys Pro Met Asn
       50                  55                  60
   Arg Tyr Ser Ala Gln Lys Gln Phe Trp Lys Ala Ala Lys Pro Gly Ala
   65                  70                  75                  80
   Asn Ser Thr Thr Asp Ala Val Leu Leu Asn Lys Leu His His Ala Ala
                   85                  90                  95
   Asp Leu Glu Lys Lys Gln Asn Glu Thr Glu Asn Arg Lys Leu Leu Gly
               100                 105                 110
   Thr Val Ile Gln Tyr Gly Asn Val Ile Gln Leu Leu His Leu Lys Ser
           115                 120                 125
   Asn Lys Tyr Leu Thr Val Asn Lys Arg Leu Pro Ala Leu Leu Glu Lys
       130                 135                 140
   Asn Ala Met Arg Val Thr Leu Asp Glu Ala Gly Asn Glu Gly Ser Trp
   145                 150                 155                 160
   Phe Tyr Ile Gln Pro Phe Tyr Lys Leu Arg Ser Ile Gly Asp Ser Val
                   165                 170                 175
   Val Ile Gly Asp Lys Val Val Leu Asn Pro Val Asn Ala Gly Gln Pro
               180                 185                 190
   Leu His Ala Ser Ser His Gln Leu Val Asp Asn Pro Gly Cys Asn Glu
           195                 200                 205
   Val Asn Ser Val Asn Cys Asn Thr Ser Trp Lys Ile Val Leu Phe Met
       210                 215                 220
   Lys Trp Ser Asp Asn Lys Asp Asp Ile Leu Lys Gly Gly Asp Val Val
   225                 230                 235                 240
   Arg Leu Phe His Ala Glu Gln Glu Lys Phe Leu Thr Cys Asp Glu His
                   245                 250                 255
   Arg Lys Lys Gln His Val Phe Leu Arg Thr Thr Gly Arg Gln Ser Ala
               260                 265                 270
   Thr Ser Ala Thr Ser Ser Lys Ala Leu Trp Glu Val Glu Val Val Gln
           275                 280                 285
   His Asp Pro Cys Arg Gly Gly Ala Gly Tyr Trp Asn Ser Leu Phe Arg
       290                 295                 300
   Phe Lys His Leu Ala Thr Gly His Tyr Leu Ala Ala Glu Val Asp Pro
   305                 310                 315                 320
   Asp Gln Asp Ala Ser Arg Ser Arg Leu Arg Asn Ala Gln Glu Lys Met
                   325                 330                 335
   Val Tyr Ser Leu Val Ser Val Pro Glu Gly Asn Asp Ile Ser Ser Ile
               340                 345                 350
   Phe Glu Leu Asp Pro Thr Thr Leu Arg Gly Gly Asp Ser Leu Val Pro
           355                 360                 365
   Arg Asn Ser Tyr Val Arg Leu Arg His Leu Cys Thr Asn Thr Trp Val
       370                 375                 380
   His Ser Thr Asn Ile Pro Ile Asp Lys Glu Glu Glu Lys Pro Val Met
   385                 390                 395                 400
   Leu Lys Ile Gly Thr Ser Pro Val Lys Glu Asp Lys Glu Ala Phe Ala
                   405                 410                 415
   Ile Val Pro Val Ser Pro Ala Glu Val Arg Asp Leu Asp Phe Ala Asn
               420                 425                 430
   Asp Ala Ser Lys Val Leu Gly Ser Ile Ala Gly Lys Leu Glu Lys Gly
           435                 440                 445
   Thr Ile Thr Gln Asn Glu Arg Arg Ser Val Thr Lys Leu Leu Glu Asp
       450                 455                 460
   Leu Val Tyr Phe Val Thr Gly Gly Thr Asn Ser Gly Gln Asp Val Leu
   465                 470                 475                 480
   Glu Val Val Phe Ser Lys Pro Asn Arg Glu Arg Gln Lys Leu Met Arg
                   485                 490                 495
   Glu Gln Asn Ile Leu Lys Gln Ile Phe Lys Leu Leu Gln Ala Pro Phe
               500                 505                 510
   Thr Asp Cys Gly Asp Gly Pro Met Leu Arg Leu Glu Glu Leu Gly Asp
           515                 520                 525
   Gln Arg His Ala Pro Phe Arg His Ile Cys Arg Leu Cys Tyr Arg Val
       530                 535                 540
   Leu Arg His Ser Gln Gln Asp Tyr Arg Lys Asn Gln Glu Tyr Ile Ala
   545                 550                 555                 560
   Lys Gln Phe Gly Phe Met Gln Lys Gln Ile Gly Tyr Asp Val Leu Ala
                   565                 570                 575
   Glu Asp Thr Ile Thr Ala Leu Leu His Asn Asn Arg Lys Leu Leu Glu
               580                 585                 590
   Lys His Ile Thr Ala Ala Glu Ile Asp Thr Phe Val Ser Leu Val Arg
           595                 600                 605
   Lys Asn Arg Glu Pro Arg Phe Leu Asp Tyr Leu Ser Asp Leu Cys Val
       610                 615                 620
   Ser Met Asn Lys Ser Ile Pro Val Thr Gln Glu Leu Ile Cys Lys Ala
   625                 630                 635                 640
   Val Leu Asn Pro Thr Asn Ala Asp Ile Leu Ile Glu Thr Lys Leu Val
                   645                 650                 655
   Leu Ser Arg Phe Glu Phe Glu Gly Val Ser Ser Thr Gly Glu Asn Ala
               660                 665                 670
   Leu Glu Ala Gly Glu Asp Glu Glu Glu Val Trp Leu Phe Trp Arg Asp
           675                 680                 685
   Ser Asn Lys Glu Ile Arg Ser Lys Ser Val Arg Glu Leu Ala Gln Asp
       690                 695                 700
   Ala Lys Glu Gly Gln Lys Glu Asp Arg Asp Val Leu Ser Tyr Tyr Arg
   705                 710                 715                 720
   Tyr Gln Leu Asn Leu Phe Ala Arg Met Cys Leu Asp Arg Gln Tyr Leu
                   725                 730                 735
   Ala Ile Asn Glu Ile Ser Gly Gln Leu Asp Val Asp Leu Ile Leu Arg
               740                 745                 750
   Cys Met Ser Asp Glu Asn Leu Pro Tyr Asp Leu Arg Ala Ser Phe Cys
           755                 760                 765
   Arg Leu Met Leu His Met His Val Asp Arg Asp Pro Gln Glu Gln Val
       770                 775                 780
   Thr Pro Val Lys Tyr Ala Arg Leu Trp Ser Glu Ile Pro Ser Glu Ile
   785                 790                 795                 800
   Ala Ile Asp Asp Tyr Asp Ser Ser Gly Ala Ser Lys Asp Glu Ile Lys
                   805                 810                 815
   Glu Arg Phe Ala Gln Thr Met Glu Phe Val Glu Glu Tyr Leu Arg Asp
               820                 825                 830
   Val Val Cys Gln Arg Phe Pro Phe Ser Asp Lys Glu Lys Asn Lys Leu
           835                 840                 845
   Thr Phe Glu Val Val Asn Leu Ala Arg Asn Leu Ile Tyr Phe Gly Phe
       850                 855                 860
   Tyr Asn Phe Ser Asp Leu Leu Arg Leu Thr Lys Ile Leu Leu Ala Ile
   865                 870                 875                 880
   Leu Asp Cys Val His Val Thr Thr Ile Phe Pro Ile Ser Lys Met Ala
                   885                 890                 895
   Lys Gly Glu Glu Asn Lys Gly Ser Asn Val Met Arg Ser Ile His Gly
               900                 905                 910
   Val Gly Glu Leu Met Thr Gln Val Val Leu Arg Gly Gly Gly Phe Leu
           915                 920                 925
   Pro Met Thr Pro Met Ala Ala Ala Pro Glu Gly Asn Val Lys Gln Ala
       930                 935                 940
   Glu Pro Glu Lys Glu Asp Ile Met Val Met Asp Thr Lys Leu Lys Ile
   945                 950                 955                 960
   Ile Glu Ile Leu Gln Phe Ile Leu Asn Val Arg Leu Asp Tyr Arg Ile
                   965                 970                 975
   Ser Cys Leu Leu Cys Ile Phe Lys Arg Glu Phe Asp Glu Ser Asn Ser
               980                 985                 990
   Gln Thr Ser Glu Thr Ser Ser Gly Asn Ser Ser Gln Glu Gly Pro Ser
           995                 1000                1005
   Asn Val Pro Gly Ala Leu Asp Phe Glu His Ile Glu Glu Gln Ala Glu
       1010                1015                1020
   Gly Ile Phe Gly Gly Ser Glu Glu Asn Thr Pro Leu Asp Leu Asp Asp
   1025                1030                1035                1040
   His Gly Gly Arg Thr Phe Leu Arg Val Leu Leu His Leu Thr Met His
                   1045                1050                1055
   Asp Tyr Pro Pro Leu Val Ser Gly Ala Leu Gln Leu Leu Phe Arg His
               1060                1065                1070
   Phe Ser Gln Arg Gln Glu Val Leu Gln Ala Phe Lys Gln Val Gln Leu
           1075                1080                1085
   Leu Val Thr Ser Gln Asp Val Asp Asn Tyr Lys Gln Ile Lys Gln Asp
       1090                1095                1100
   Leu Asp Gln Leu Arg Ser Ile Val Glu Lys Ser Glu Leu Trp Val Tyr
   1105                1110                1115                1120
   Lys Gly Gln Gly Pro Asp Glu Thr Met Asp Gly Ala Ser Gly Glu Asn
                   1125                1130                1135
   Glu His Lys Lys Thr Glu Glu Gly Asn Asn Lys Pro Gln Lys His Glu
               1140                1145                1150
   Ser Thr Ser Ser Tyr Asn Tyr Arg Val Val Lys Glu Ile Leu Ile Arg
           1155                1160                1165
   Leu Ser Lys Leu Cys Val Gln Glu Ser Ala Ser Val Arg Lys Ser Arg
       1170                1175                1180
   Lys Gln Gln Gln Arg Leu Leu Arg Asn Met Gly Ala His Ala Val Val
   1185                1190                1195                1200
   Leu Glu Leu Leu Gln Ile Pro Tyr Glu Lys Ala Glu Asp Thr Lys Met
                   1205                1210                1215
   Gln Glu Ile Met Arg Leu Ala His Glu Phe Leu Gln Asn Phe Cys Ala
               1220                1225                1230
   Gly Asn Gln Gln Asn Gln Ala Leu Leu His Lys His Ile Asn Leu Phe
           1235                1240                1245
   Leu Asn Pro
       1250
<210> 3
   <211> 2282
   <212> PRT
   <213> Artificial Sequence
<220>
   <223> ITPR fragment
<400> 3
   Met Ser Asp Lys Met Ser Ser Phe Leu His Ile Gly Asp Ile Cys Ser
   1               5                   10                  15
   Leu Tyr Ala Glu Gly Ser Thr Asn Gly Phe Ile Ser Thr Leu Gly Leu
               20                  25                  30
   Val Asp Asp Arg Cys Val Val Gln Pro Glu Thr Gly Asp Leu Asn Asn
           35                  40                  45
   Pro Pro Lys Lys Phe Arg Asp Cys Leu Phe Lys Leu Cys Pro Met Asn
       50                  55                  60
   Arg Tyr Ser Ala Gln Lys Gln Phe Trp Lys Ala Ala Lys Pro Gly Ala
   65                  70                  75                  80
   Asn Ser Thr Thr Asp Ala Val Leu Leu Asn Lys Leu His His Ala Ala
                   85                  90                  95
   Asp Leu Glu Lys Lys Gln Asn Glu Thr Glu Asn Arg Lys Leu Leu Gly
               100                 105                 110
   Thr Val Ile Gln Tyr Gly Asn Val Ile Gln Leu Leu His Leu Lys Ser
           115                 120                 125
   Asn Lys Tyr Leu Thr Val Asn Lys Arg Leu Pro Ala Leu Leu Glu Lys
       130                 135                 140
   Asn Ala Met Arg Val Thr Leu Asp Glu Ala Gly Asn Glu Gly Ser Trp
   145                 150                 155                 160
   Phe Tyr Ile Gln Pro Phe Tyr Lys Leu Arg Ser Ile Gly Asp Ser Val
                   165                 170                 175
   Val Ile Gly Asp Lys Val Val Leu Asn Pro Val Asn Ala Gly Gln Pro
               180                 185                 190
   Leu His Ala Ser Ser His Gln Leu Val Asp Asn Pro Gly Cys Asn Glu
           195                 200                 205
   Val Asn Ser Val Asn Cys Asn Thr Ser Trp Lys Ile Val Leu Phe Met
       210                 215                 220
   Lys Trp Ser Asp Asn Lys Asp Asp Ile Leu Lys Gly Gly Asp Val Val
   225                 230                 235                 240
   Arg Leu Phe His Ala Glu Gln Glu Lys Phe Leu Thr Cys Asp Glu His
                   245                 250                 255
   Arg Lys Lys Gln His Val Phe Leu Arg Thr Thr Gly Arg Gln Ser Ala
               260                 265                 270
   Thr Ser Ala Thr Ser Ser Lys Ala Leu Trp Glu Val Glu Val Val Gln
           275                 280                 285
   His Asp Pro Cys Arg Gly Gly Ala Gly Tyr Trp Asn Ser Leu Phe Arg
       290                 295                 300
   Phe Lys His Leu Ala Thr Gly His Tyr Leu Ala Ala Glu Val Asp Pro
   305                 310                 315                 320
   Asp Gln Asp Ala Ser Arg Ser Arg Leu Arg Asn Ala Gln Glu Lys Met
                   325                 330                 335
   Val Tyr Ser Leu Val Ser Val Pro Glu Gly Asn Asp Ile Ser Ser Ile
               340                 345                 350
   Phe Glu Leu Asp Pro Thr Thr Leu Arg Gly Gly Asp Ser Leu Val Pro
           355                 360                 365
   Arg Asn Ser Tyr Val Arg Leu Arg His Leu Cys Thr Asn Thr Trp Val
       370                 375                 380
   His Ser Thr Asn Ile Pro Ile Asp Lys Glu Glu Glu Lys Pro Val Met
   385                 390                 395                 400
   Leu Lys Ile Gly Thr Ser Pro Val Lys Glu Asp Lys Glu Ala Phe Ala
                   405                 410                 415
   Ile Val Pro Val Ser Pro Ala Glu Val Arg Asp Leu Asp Phe Ala Asn
               420                 425                 430
   Asp Ala Ser Lys Val Leu Gly Ser Ile Ala Gly Lys Leu Glu Lys Gly
           435                 440                 445
   Thr Ile Thr Gln Asn Glu Arg Arg Ser Val Thr Lys Leu Leu Glu Asp
       450                 455                 460
   Leu Val Tyr Phe Val Thr Gly Gly Thr Asn Ser Gly Gln Asp Val Leu
   465                 470                 475                 480
   Glu Val Val Phe Ser Lys Pro Asn Arg Glu Arg Gln Lys Leu Met Arg
                   485                 490                 495
   Glu Gln Asn Ile Leu Lys Gln Ile Phe Lys Leu Leu Gln Ala Pro Phe
               500                 505                 510
   Thr Asp Cys Gly Asp Gly Pro Met Leu Arg Leu Glu Glu Leu Gly Asp
           515                 520                 525
   Gln Arg His Ala Pro Phe Arg His Ile Cys Arg Leu Cys Tyr Arg Val
       530                 535                 540
   Leu Arg His Ser Gln Gln Asp Tyr Arg Lys Asn Gln Glu Tyr Ile Ala
   545                 550                 555                 560
   Lys Gln Phe Gly Phe Met Gln Lys Gln Ile Gly Tyr Asp Val Leu Ala
                   565                 570                 575
   Glu Asp Thr Ile Thr Ala Leu Leu His Asn Asn Arg Lys Leu Leu Glu
               580                 585                 590
   Lys His Ile Thr Ala Ala Glu Ile Asp Thr Phe Val Ser Leu Val Arg
           595                 600                 605
   Lys Asn Arg Glu Pro Arg Phe Leu Asp Tyr Leu Ser Asp Leu Cys Val
       610                 615                 620
   Ser Met Asn Lys Ser Ile Pro Val Thr Gln Glu Leu Ile Cys Lys Ala
   625                 630                 635                 640
   Val Leu Asn Pro Thr Asn Ala Asp Ile Leu Ile Glu Thr Lys Leu Val
                   645                 650                 655
   Leu Ser Arg Phe Glu Phe Glu Gly Val Ser Ser Thr Gly Glu Asn Ala
               660                 665                 670
   Leu Glu Ala Gly Glu Asp Glu Glu Glu Val Trp Leu Phe Trp Arg Asp
           675                 680                 685
   Ser Asn Lys Glu Ile Arg Ser Lys Ser Val Arg Glu Leu Ala Gln Asp
       690                 695                 700
   Ala Lys Glu Gly Gln Lys Glu Asp Arg Asp Val Leu Ser Tyr Tyr Arg
   705                 710                 715                 720
   Tyr Gln Leu Asn Leu Phe Ala Arg Met Cys Leu Asp Arg Gln Tyr Leu
                   725                 730                 735
   Ala Ile Asn Glu Ile Ser Gly Gln Leu Asp Val Asp Leu Ile Leu Arg
               740                 745                 750
   Cys Met Ser Asp Glu Asn Leu Pro Tyr Asp Leu Arg Ala Ser Phe Cys
           755                 760                 765
   Arg Leu Met Leu His Met His Val Asp Arg Asp Pro Gln Glu Gln Val
       770                 775                 780
   Thr Pro Val Lys Tyr Ala Arg Leu Trp Ser Glu Ile Pro Ser Glu Ile
   785                 790                 795                 800
   Ala Ile Asp Asp Tyr Asp Ser Ser Gly Ala Ser Lys Asp Glu Ile Lys
                   805                 810                 815
   Glu Arg Phe Ala Gln Thr Met Glu Phe Val Glu Glu Tyr Leu Arg Asp
               820                 825                 830
   Val Val Cys Gln Arg Phe Pro Phe Ser Asp Lys Glu Lys Asn Lys Leu
           835                 840                 845
   Thr Phe Glu Val Val Asn Leu Ala Arg Asn Leu Ile Tyr Phe Gly Phe
       850                 855                 860
   Tyr Asn Phe Ser Asp Leu Leu Arg Leu Thr Lys Ile Leu Leu Ala Ile
   865                 870                 875                 880
   Leu Asp Cys Val His Val Thr Thr Ile Phe Pro Ile Ser Lys Met Ala
                   885                 890                 895
   Lys Gly Glu Glu Asn Lys Gly Ser Asn Val Met Arg Ser Ile His Gly
               900                 905                 910
   Val Gly Glu Leu Met Thr Gln Val Val Leu Arg Gly Gly Gly Phe Leu
           915                 920                 925
   Pro Met Thr Pro Met Ala Ala Ala Pro Glu Gly Asn Val Lys Gln Ala
       930                 935                 940
   Glu Pro Glu Lys Glu Asp Ile Met Val Met Asp Thr Lys Leu Lys Ile
   945                 950                 955                 960
   Ile Glu Ile Leu Gln Phe Ile Leu Asn Val Arg Leu Asp Tyr Arg Ile
                   965                 970                 975
   Ser Cys Leu Leu Cys Ile Phe Lys Arg Glu Phe Asp Glu Ser Asn Ser
               980                 985                 990
   Gln Thr Ser Glu Thr Ser Ser Gly Asn Ser Ser Gln Glu Gly Pro Ser
           995                 1000                1005
   Asn Val Pro Gly Ala Leu Asp Phe Glu His Ile Glu Glu Gln Ala Glu
       1010                1015                1020
   Gly Ile Phe Gly Gly Ser Glu Glu Asn Thr Pro Leu Asp Leu Asp Asp
   1025                1030                1035                1040
   His Gly Gly Arg Thr Phe Leu Arg Val Leu Leu His Leu Thr Met His
                   1045                1050                1055
   Asp Tyr Pro Pro Leu Val Ser Gly Ala Leu Gln Leu Leu Phe Arg His
               1060                1065                1070
   Phe Ser Gln Arg Gln Glu Val Leu Gln Ala Phe Lys Gln Val Gln Leu
           1075                1080                1085
   Leu Val Thr Ser Gln Asp Val Asp Asn Tyr Lys Gln Ile Lys Gln Asp
       1090                1095                1100
   Leu Asp Gln Leu Arg Ser Ile Val Glu Lys Ser Glu Leu Trp Val Tyr
   1105                1110                1115                1120
   Lys Gly Gln Gly Pro Asp Glu Thr Met Asp Gly Ala Ser Gly Glu Asn
                   1125                1130                1135
   Glu His Lys Lys Thr Glu Glu Gly Asn Asn Lys Pro Gln Lys His Glu
               1140                1145                1150
   Ser Thr Ser Ser Tyr Asn Tyr Arg Val Val Lys Glu Ile Leu Ile Arg
           1155                1160                1165
   Leu Ser Lys Leu Cys Val Gln Glu Ser Ala Ser Val Arg Lys Ser Arg
       1170                1175                1180
   Lys Gln Gln Gln Arg Leu Leu Arg Asn Met Gly Ala His Ala Val Val
   1185                1190                1195                1200
   Leu Glu Leu Leu Gln Ile Pro Tyr Glu Lys Ala Glu Asp Thr Lys Met
                   1205                1210                1215
   Gln Glu Ile Met Arg Leu Ala His Glu Phe Leu Gln Asn Phe Cys Ala
               1220                1225                1230
   Gly Asn Gln Gln Asn Gln Ala Leu Leu His Lys His Ile Asn Leu Phe
           1235                1240                1245
   Leu Asn Pro Gly Ile Leu Glu Ala Val Thr Met Gln His Ile Phe Met
       1250                1255                1260
   Asn Asn Phe Gln Leu Cys Ser Glu Ile Asn Glu Arg Val Val Gln His
   1265                1270                1275                1280
   Phe Val His Cys Ile Glu Thr His Gly Arg Asn Val Gln Tyr Ile Lys
                   1285                1290                1295
   Phe Leu Gln Thr Ile Val Lys Ala Glu Gly Lys Phe Ile Lys Lys Cys
               1300                1305                1310
   Gln Asp Met Val Met Ala Glu Leu Val Asn Ser Gly Glu Asp Val Leu
           1315                1320                1325
   Val Phe Tyr Asn Asp Arg Ala Ser Phe Gln Thr Leu Ile Gln Met Met
       1330                1335                1340
   Arg Ser Glu Arg Asp Arg Met Asp Glu Asn Ser Pro Leu Met Tyr His
   1345                1350                1355                1360
   Ile His Leu Val Glu Leu Leu Ala Val Cys Thr Glu Gly Lys Asn Val
                   1365                1370                1375
   Tyr Thr Glu Ile Lys Cys Asn Ser Leu Leu Pro Leu Asp Asp Ile Val
               1380                1385                1390
   Arg Val Val Thr His Glu Asp Cys Ile Pro Glu Val Lys Ile Ala Tyr
           1395                1400                1405
   Ile Asn Phe Leu Asn His Cys Tyr Val Asp Thr Glu Val Glu Met Lys
       1410                1415                1420
   Glu Ile Tyr Thr Ser Asn His Met Trp Lys Leu Phe Glu Asn Phe Leu
   1425                1430                1435                1440
   Val Asp Ile Cys Arg Ala Cys Asn Asn Thr Ser Asp Arg Lys His Ala
                   1445                1450                1455
   Asp Ser Ile Leu Glu Lys Tyr Val Thr Glu Ile Val Met Ser Ile Val
               1460                1465                1470
   Thr Thr Phe Phe Ser Ser Pro Phe Ser Asp Gln Ser Thr Thr Leu Gln
           1475                1480                1485
   Thr Arg Gln Pro Val Phe Val Gln Leu Leu Gln Gly Val Phe Arg Val
       1490                1495                1500
   Tyr His Cys Asn Trp Leu Met Pro Ser Gln Lys Ala Ser Val Glu Ser
   1505                1510                1515                1520
   Cys Ile Arg Val Leu Ser Asp Val Ala Lys Ser Arg Ala Ile Ala Ile
                   1525                1530                1535
   Pro Val Asp Leu Asp Ser Gln Val Asn Asn Leu Phe Leu Lys Ser His
               1540                1545                1550
   Ser Ile Val Gln Lys Thr Ala Met Asn Trp Arg Leu Ser Ala Arg Asn
           1555                1560                1565
   Ala Ala Arg Arg Asp Ser Val Leu Ala Ala Ser Arg Asp Tyr Arg Asn
       1570                1575                1580
   Ile Ile Glu Arg Leu Gln Asp Ile Val Ser Ala Leu Glu Asp Arg Leu
   1585                1590                1595                1600
   Arg Pro Leu Val Gln Ala Glu Leu Ser Val Leu Val Asp Val Leu His
                   1605                1610                1615
   Arg Pro Glu Leu Leu Phe Pro Glu Asn Thr Asp Ala Arg Arg Lys Cys
               1620                1625                1630
   Glu Ser Gly Gly Phe Ile Cys Lys Leu Ile Lys His Thr Lys Gln Leu
           1635                1640                1645
   Leu Glu Glu Asn Glu Glu Lys Leu Cys Ile Lys Val Leu Gln Thr Leu
       1650                1655                1660
   Arg Glu Met Met Thr Lys Asp Arg Gly Tyr Gly Glu Lys Gly Glu Ala
   1665                1670                1675                1680
   Leu Arg Gln Val Leu Val Asn Arg Tyr Tyr Gly Asn Val Arg Pro Ser
                   1685                1690                1695
   Gly Arg Arg Glu Ser Leu Thr Ser Phe Gly Asn Gly Pro Leu Ser Ala
               1700                1705                1710
   Gly Gly Pro Gly Lys Pro Gly Gly Gly Gly Gly Gly Ser Gly Ser Ser
           1715                1720                1725
   Ser Met Ser Arg Gly Glu Met Ser Leu Ala Glu Val Gln Cys His Leu
       1730                1735                1740
   Asp Lys Glu Gly Ala Ser Asn Leu Val Ile Asp Leu Ile Met Asn Ala
   1745                1750                1755                1760
   Ser Ser Asp Arg Val Phe His Glu Ser Ile Leu Leu Ala Ile Ala Leu
                   1765                1770                1775
   Leu Glu Gly Gly Asn Thr Thr Ile Gln His Ser Phe Phe Cys Arg Leu
               1780                1785                1790
   Thr Glu Asp Lys Lys Ser Glu Lys Phe Phe Lys Val Phe Tyr Asp Arg
           1795                1800                1805
   Met Lys Val Ala Gln Gln Glu Ile Lys Ala Thr Val Thr Val Asn Thr
       1810                1815                1820
   Ser Asp Leu Gly Asn Lys Lys Lys Asp Asp Glu Val Asp Arg Asp Ala
   1825                1830                1835                1840
   Pro Ser Arg Lys Lys Ala Lys Glu Pro Thr Thr Gln Ile Thr Glu Glu
                   1845                1850                1855
   Val Arg Asp Gln Leu Leu Glu Ala Ser Ala Ala Thr Arg Lys Ala Phe
               1860                1865                1870
   Thr Thr Phe Arg Arg Glu Ala Asp Pro Asp Asp His Tyr Gln Pro Gly
           1875                1880                1885
   Glu Gly Thr Gln Ala Thr Ala Asp Lys Ala Lys Asp Asp Leu Glu Met
       1890                1895                1900
   Ser Ala Val Ile Thr Ile Met Gln Pro Ile Leu Arg Phe Leu Gln Leu
   1905                1910                1915                1920
   Leu Cys Glu Asn His Asn Arg Asp Leu Gln Asn Phe Leu Arg Cys Gln
                   1925                1930                1935
   Asn Asn Lys Thr Asn Tyr Asn Leu Val Cys Glu Thr Leu Gln Phe Leu
               1940                1945                1950
   Asp Cys Ile Cys Gly Ser Thr Thr Gly Gly Leu Gly Leu Leu Gly Leu
           1955                1960                1965
   Tyr Ile Asn Glu Lys Asn Val Ala Leu Ile Asn Gln Thr Leu Glu Ser
       1970                1975                1980
   Leu Thr Glu Tyr Cys Gln Gly Pro Cys His Glu Asn Gln Asn Cys Ile
   1985                1990                1995                2000
   Ala Thr His Glu Ser Asn Gly Ile Asp Ile Ile Thr Ala Leu Ile Leu
                   2005                2010                2015
   Asn Asp Ile Asn Pro Leu Gly Lys Lys Arg Met Asp Leu Val Leu Glu
               2020                2025                2030
   Leu Lys Asn Asn Ala Ser Lys Leu Leu Leu Ala Ile Met Glu Ser Arg
           2035                2040                2045
   His Asp Ser Glu Asn Ala Glu Arg Ile Leu Tyr Asn Met Arg Pro Lys
       2050                2055                2060
   Glu Leu Val Glu Val Ile Lys Lys Ala Tyr Met Gln Gly Glu Val Glu
   2065                2070                2075                2080
   Phe Glu Asp Gly Glu Asn Gly Glu Asp Gly Ala Ala Ser Pro Arg Asn
                   2085                2090                2095
   Val Gly His Asn Ile Tyr Ile Leu Ala His Gln Leu Ala Arg His Asn
               2100                2105                2110
   Lys Glu Leu Gln Ser Met Leu Lys Pro Gly Gly Gln Val Asp Gly Asp
           2115                2120                2125
   Glu Ala Leu Glu Phe Tyr Ala Lys His Thr Ala Gln Ile Glu Ile Val
       2130                2135                2140
   Arg Leu Asp Arg Thr Met Glu Gln Ile Val Phe Pro Val Pro Ser Ile
   2145                2150                2155                2160
   Cys Glu Phe Leu Thr Lys Glu Ser Lys Leu Arg Ile Tyr Tyr Thr Thr
                   2165                2170                2175
   Glu Arg Asp Glu Gln Gly Ser Lys Ile Asn Asp Phe Phe Leu Arg Ser
               2180                2185                2190
   Glu Asp Leu Phe Asn Glu Met Asn Trp Gln Lys Lys Leu Arg Ala Gln
           2195                2200                2205
   Pro Val Leu Tyr Trp Cys Ala Arg Asn Met Ser Phe Trp Ser Ser Ile
       2210                2215                2220
   Ser Phe Asn Leu Ala Val Leu Met Asn Leu Leu Val Ala Phe Phe Tyr
   2225                2230                2235                2240
   Pro Phe Lys Gly Val Arg Gly Gly Thr Leu Glu Pro His Trp Ser Gly
                   2245                2250                2255
   Leu Leu Trp Thr Ala Met Leu Ile Ser Leu Ala Ile Val Ile Ala Leu
               2260                2265                2270
   Pro Lys Pro His Gly Ile Arg Ala Leu Ile
           2275                2280

## Description

The present invention relates to an *in vitro* method for diagnosing a disease according to claim 1, comprising the step detecting in a sample from a patient an autoantibody binding to ITPR1 data base code Q14643; use of a polypeptide comprising amino acids 1-1251 of ITPR1 data base code Q14643 or a variant having at least 98 percent sequence identity with ITPR1 as defined by the Uniprot data base code Q14643 for the *in vitro* diagnosis of a disease according to claim 3, comprising the step detecting autoantibodies binding to ITPR1 data base code Q14643; use of an autoantibody, preferably an isolated autoantibody, binding to polypeptide ITPR1 data base code Q14643, wherein the autoantibody is preferably in complex with said polypeptide, wherein the disease is paraneoplastic neurological syndrome, preferably associated with breast carcinoma, and wherein an autoantibody to ITPR1 is detedted.

Developing diagnostic systems for neurological diseases is a continuing challenge in biomedical science, not in the least because many symptoms encountered may be accounted for by a huge variety of causes including genetically-inherited diseases, drug abuse, malnutrition, infection, injury, psychiatric illness, immunological defects and cancer.

Since a neurological disease is rarely associated with a characteristic pattern of clinical symptoms, it is often difficult to provide a reliable diagnosis solely based on the observation and examination of the patients affected or their medical history.

The importance of an early diagnosis cannot be overemphasized. Many neurological disorders, most prominently Alzheimer's and Parkinson's diseases, cannot be cured, but drugs are available that may be used to slow down their progression. The earlier the diagnosis, the better the chances to exploit the spectrum of available drugs to the full benefit of the patient.

This holds all the more true in the case of neurological diseases associated with autoantibodies. In some cases, the link between a specific detectable autoantibody and a condition is sufficiently strong to allow for an immediate diagnosis.

But even if it is not, the detection of autoantibodies may point the physician in charge to therapeutic means that may be used to ameliorate the patient's condition. There is a variety of widely used immunosuppressants that may be used regardless of the nature of the autoantibody's target. Alternatively, apheresis may be used to remove autoantibodies from the patient's blood. In many cases, patients went on to lead a normal life following early diagnosis and treatment of a neurological autoimmune disease.

Diagnostic assays based on the detection of autoantibodies may also corroborate the diagnosis of diseases other than those associated autoantibodies. If it turns out that a blood sample is devoid of specific autoantibodies, this is likely to help the physician in charge exclude a range of possibilities and thus narrow down the spectrum of plausible conditions.

Examples of neurological conditions coinciding with the emergence of autoantibodies include Neuromyelitis optica, a disease characterised by loss of vision and spinal cord function, and anti-NMDA receptor encephalitis, which is associated with autonomic dysfunction, hypoventilation, cerebellar ataxia, hemiparesis, loss of consciousness, or catatonia. Whilst the involvement of autoantibodies and the nature of these conditions as such was previously poorly understood, many of these disease can now be diagnosed and treated efficiently owing to the availability of assays based on the detection of autoantibodies.

Therefore, it is paramount that new approaches to distinguish neurological conditions associated with autoantibodies from others be developed.

US2010/035360 discloses that an autoantibody to ITPR1 could be found in sera from patients having Sjogrens's syndrome or rheumatoid arthritis.

WO2005/017151 discloses the expression of a mutant protein consisting of the channel domain of a IP3 receptor.

Kurien Biji T (2009) XP002737576 (Methods in Molecular Biology 2009, vol. 536, pages 201-211) discloses the affinity purification of autoantibodies from sera.

EP 2 952 898 discloses the detection of an autoantibody to human neuronal Na(+)/K(+) ATPase for the detection of neurological diseases

A problem underlying the present invention is to provide an agent and a method for diagnosing neurological disease, more specifically distinguishing diseases associated with neurological symptoms and the emergence of autoantibodies from other types of diseases.

Another problem underlying the present invention is to provide an autoantibody that, when found in a liquid sample taken from a patient, indicates that said patient is suffering from an autoimmune disease associated with neurological symptoms.

Another problem underlying the present invention is to provide an agent and a method for diagnosing and treating an autoimmune disease associated with neurological symptoms.

The problem underlying the present invention is solved by the subject-matter of the attached independent and dependent claims.

In a first aspect, the problem underlying the present invention is solved by an *in vitro* method for diagnosing a disease according to claim 1, the disease is paraneoplastic neurological syndrome, comprising the step detecting in a sample from a patient an autoantibody binding to ITPR1 base code Q14643, wherein detection of the autoantibody indicates an increased likelihood that the patient suffers from the disease.

In a preferred embodiment, the sample is a bodily fluid comprising antibodies, preferably selected from the group comprising whole-blood, serum, cerebrospinal fluid and saliva.

In a second aspect, the problem underlying the present invention is solved by a use of a polypeptide comprising amino acids 1-1251 of ITPR1 base code Q14643 or a variant thereof thereof having the ability to bind specifically to an autoantibody to ITPR1 base code Q14643 for the *in vitro* diagnosis of a disease, comprising the step detecting autoantibodies binding to ITPR1 base code Q14643, wherein the disease is paraneoplastic neurological syndrome, preferably associated with breast carcinoma, wherein the variant has at least 98 percent sequence identity with ITPR1 as defined by the Uniprot data base code Q14643.

In a third aspect, the problem underlying the present invention is solved by a use of an autoantibody, preferably an isolated autoantibody, binding to polypeptide ITPR1, wherein the autoantibody is preferably in complex with said polypeptide, for the *in vitro* diagnosis of a disease, wherein the disease is paraneoplastic neurological syndrome, preferably associated with breast carcinoma, and wherein said autoantibody to ITPR1 is detected.

In an 4th aspect, the problem underlying the present invention is solved by a use of a medical or diagnostic device comprising a polypeptide comprising amino acids 1-1251 of ITPR1 base code Q14643 or a variant thereof thereof having the ability to bind specifically to an autoantibody to ITPR1 base code Q14643 for the *in vitro* diagnosis of a disease, wherein the disease is paraneoplastic neurological syndrome, preferably associated with breast carcinoma, and wherein an autoantibody to ITPR1 base code Q14643 is detected, wherein the variant has at least 98 percent sequence identity with ITPR1 as defined by the Uniprot data base code Q14643.

In a preferred embodiment of any aspect of the present invention, the polypeptide is provided in the form of a cell comprising a nucleic acid encoding said polypeptide or in the form of a tissue comprising said polypeptide.

In a preferred embodiment of any aspect of the present invention, the polypeptide is a recombinant and/or isolated polypeptide.

The present invention is based on the inventors' surprising finding that autoantibodies to ITPR1 may be detected in samples from a number of patients suffering from neurological symptoms, but not in samples obtained from healthy subjects. The presence of such autoantibodies suggests that ITPR1 activity and function are impaired in patients having ITPR1 autoantibodies to the effect that neurological symptoms occur.

The present disclosure relates to a polypeptide comprising a human ITPR1 data base code Q14643 or variants thereof having the ability to bind specifically to an autoantibody to ITPR1 data base code Q14643 Throughout this application, any data base codes cited refer to the Uniprot data base, more specifically the version accessible on-line on October 27, 2014. D26070.1 represents a nucleotide sequence encoding ITPR1.

Inositol trisphosphate receptor type 1 (ITPR1; also referred to in the state of art as IP3R 1, IP3 receptor, type 1 InsP3 receptor, inositol 1,4,5-triphosphate receptor, type 1 protein phosphatase 1, and regulatory subunit 94 type 1 inositol 1,4,5-trisphosphate receptor) is a membrane glycoprotein complex acting as a Ca²⁺ channel following activation by inositol trisphosphate (IP3). In humans it is encoded by the ITPR1 gene (also referred to in the state of art as ACV; CLA4; IP3R; IP3R1; SCA15; SCA16; SCA29; INSP3R1; PPP1R94).

IP3-gated intracellular Ca²⁺ release channels are composed of four IP3 receptor subunits (N. Maeda,T. Kawasaki, S. Nakade, N. Yokota, T. Taguchi, M. Kasai, and K. Mikoshiba, (1991) J. Biol. Chem. 266, 1109-1116). There are at least three types of IP3 receptor (ITPR1, ITPR2, and ITPR3) (T. Furuichi, and K. Mikoshiba, (1995) J. Neurochem. 64, 953-960), and they exist both as homo- and heterotetramers (T. Monkawa, A. Miyawaki, T. Sugiyama, H. Yoneshima, M. Yamamoto-Hino, T. Furuichi, T. Saruta, M. Hasegawa, and K. Mikoshiba, (1995) J. Biol. Chem. 270,14700-14704).

At the functional level, the ITPR1 can be divided into five distinct domains: an N-terminal suppressor domain (amino acids 1-225), an IP3-binding core (amino acids 226-578), a modulatory and transducing domain (amino acids 579 -2275), a channel domain with 6 transmembrane helices (amino acids 2276-2589), and finally a C-terminal coupling domain (amino acids 2590 2749) (K. Uchida, H. Miyauchi, T. Furuichi, T. Michikawa, K. Mikoshiba, Critical regions for activation gating of the inositol 1,4,5-trisphosphate receptor, J. Biol. Chem. 278 (2003) 16551-16560). The IP3-binding core is an essential region for specific IP3 binding (F. Yoshikawa, M. Morita, T. Monkawa, T. Michikawa, T. Furuichi, and K. Mikoshiba. Mutation analysis of the ligand binding site of the inositol 1,4,5-trisphosphate receptor. (1996) J. Biol. Chem. 271, 18277--18284) and contains 11 basic amino acids essential for its activity (Bosanac et al., 2002).

Apart from a number of short loops between the transmembrane helices 1 and 2, and 3 and 4, only a larger loop comprising 106 amino acids that is located between transmembrane helices 5 and 6 is in contact with the ER lumen. This loop consists of a variable region (66 amino acids) containing two N-glycosylation sites followed by a conserved region (40 amino acids) contributing to the channel pore.

IP3-induced Ca²⁺ release is a positively cooperative process (T. Meyer, T. Wensel, and L. Stryer, Kinetics of calcium channel opening by inositol 1,4,5-trisphosphate. (1990) Biochemistry 29, 32--37.; J. Hirota, T. Michikawa, A. Miyawaki, T. Furuichi, I. Okura, and K. Mikoshiba, Kinetics of calcium release by immunoaffinity-purified inositol 1,4,5-trisphosphate receptor in reconstituted lipid vesicles. (1995) J. Biol. Chem. 270, 19046-19051; T. Michikawa, J. Hirota, S. Kawano, M. Hiraoka, M. Yamada, T. Furuichi, and K. Mikoshiba. Calmodulin mediates calcium-dependent inactivation of the cerebellar type 1 inositol 1,4,5-trisphosphate receptor. Neuron 23 (1999), 799-808), i.e. the binding of at least two IP3 molecules to a single tetrameric IP3 receptor channel, is required for channel opening. ITPR1 is activated by IP3, wherein its affinity to IP3 is lower than ITPR2's, but higher than ITPR3's (C.L. Newton, G.A. Mignery, T.C. Südhof, Co-expression in vertebrate tissues and cell lines of multiple inositol 1,4,5-trisphosphate (InsP3) receptors with distinct affinities for InsP3, J. Biol. Chem. 269 (1994) 28613-28619); Missiaen et al., 1998; Miyakawa et al., 1998).

In addition to IP3, cytosolic Ca²⁺ functions as a co-agonist. Importantly, Ca²⁺ activates the IP3 receptor at low concentrations, typically below 300 nM, while it inhibits IP3 receptor at higher concentrations (Lino, 1990; E.A. Finch, T.J. Turner, S.M. Goldin, Calcium as a coagonist of inositol 1,4,5-trisphosphate-induced calcium release, Science 252 (1991) 443-446.; Bezprozvanny et al., 1991; J.B. Parys, S.W. Sernett, S. DeLisle, P.M. Snyder, M.J. Welsh, K.P. Campbell, Isolation, characterization, and localization of the inositol 1,4,5-trisphosphate receptor protein in Xenopus laevis oocytes, J. Biol. Chem. 267 (1992) 18776-18782.).

Luminal Ca²⁺ is also deemed important, and the depletion of the Ca²⁺ stores leads to a decreased IP3 receptor sensitivity (L. Missiaen, C.W. Taylor, M.J. Berridge, Spontaneous calcium release from inositol trisphosphate-sensitive calcium stores, Nature 352 (1991) 241-244; L. Missiaen, H. De Smedt, G. Droogmans, R. Casteels, Ca2+ release induced by inositol 1,4,5-trisphosphate is a steady-state phenomenon controlled by luminal Ca2+ in permeabilized cells, Nature 357 (1992) 599-602;; D.L. Nunn, C.W. Taylor, Luminal Ca2+ increases the sensitivity of Ca2+ stores to inositol 1,4,5-trisphosphate, Mol. Pharmacol. 41 (1992) 115-119.; J.B. Parys, L. Missiaen, H. De Smedt, R. Casteels, Loading dependence of inositol 1,4,5-trisphosphate-induced Ca2+ release in the clonal cell line A7r5. Implications for the mechanism of quantal Ca2+ release, J. Biol. Chem. 268 (1993) 25206-25212.).

Modulation of IP3 receptor activity occurs by three main mechanisms: (1) the local environment, including pH, ATP and Mg2+ concentration and redox status; (2) its phosphorylation status, which depends on the activity of many different kinases and phosphatases, some of them forming a complex with the ITPR1; (3) regulatory proteins that directly affect IP3 receptor activity by either a stimulatory or an inhibitory way (Mikoshiba, 2007; J.B. Parys, H. De Smedt, Inositol 1,4,5-trisphosphate and its receptors, Adv. Exp. Med. Biol. 740 (2012) 255-280; V. Vanderheyden, B. Devogelaere, L. Missiaen, H. De Smedt, G. Bultynck, J.B. Parys, Regulation of inositol 1,4,5-trisphosphate-induced Ca2+ release by reversible phosphorylation and dephosphorylation, Biochim. Biophys. Acta 1793 (2009) 959-970).

The teachings of the present disclosure may not only be carried out using polypeptides, in particular a polypeptide comprising ITPR1 as encoded by the data base code Q14643, or nucleic acids having the exact sequences referred to in this application explicitly, for example by function, name, sequence or accession number, or implicitly, but also using variants of such polypeptides or nucleic acids.

The term "variant", as used herein, may refer to at least one fragment of the full length sequence referred to, more specifically one or more amino acid or nucleic acid sequence which is, relative to the full-length sequence, truncated at one or both termini by one or more amino acids. In a preferred embodiment, the fragment comprises amino acids 1-2282.The fragment comprises amino acids 1-1251 of ITPR1 base code Q14643.

The term "variant" relates not only to at least one fragment, but also to a polypeptide or a fragment thereof comprising amino acid sequences that are at least 98 or 99 % identical to the reference amino acid sequence referred to or the fragment thereof, wherein amino acids other than those essential for the biological activity, for example the ability of an antigen to bind to an (auto)antibody, or the fold or structure of the polypeptide are deleted or substituted and/or one or more such essential amino acids are replaced in a conservative manner and/or amino acids are added such that the biological activity of the polypeptide is preserved. The state of the art comprises various methods that may be used to align two given nucleic acid or amino acid sequences and to calculate the degree of identity, see for example Arthur Lesk (2008), Introduction to bioinformatics, Oxford University Press, 2008, 3^{rd} edition. In a preferred embodiment, the ClustalW software (Larkin, M. A., Blackshields, G., Brown, N. P., Chenna, R., McGettigan, P. A., McWilliam, H., Valentin, F., Wallace, I. M., Wilm, A., Lopez, R., Thompson, J. D., Gibson, T. J., Higgins, D. G. (2007). Clustal W and Clustal X version 2.0. Bioinformatics, 23, 2947-2948) is used using default settings.

Variants may, in addition, comprise chemical modifications, for example isotopic labels or covalent modifications such as glycosylation, phosphorylation, acetylation, decarboxylation, citrullination, hydroxylation and the like. The person skilled in the art is familiar with methods to modify polypeptides. Any modification is designed such that it does not abolish the biological activity of the variant.

Moreover, variants may also be generated by fusion with other known polypeptides or variants thereof and comprise active portions or domains, preferably having a sequence identity of at least 98 or 99 % when aligned with the active portion of the reference sequence, wherein the term "active portion", as used herein, refers to an amino acid sequence, which is less than the full length amino acid sequence or, in the case of a nucleic acid sequence, codes for less than the full length amino acid sequence, respectively, and/or is a variant of the natural sequence, but retains at least some of the biological activity.

The term "variant" of a nucleic acid comprises nucleic acids the complementary strand of which hybridizes, preferably under stringent conditions, to the reference or wild type nucleic acid. Stringency of hybridization reactions is readily determinable by one of ordinary skilled in the art, and in general is an empirical calculation dependent on probe length, washing temperature and salt concentration. In general longer probes require higher temperatures for proper annealing, while shorter probes less so. Hybridization generally depends on the ability of denatured DNA to reanneal to complementary strands present in an environment below their melting temperature: The higher the degree of desired homology between the probe and hybridizable sequence, the higher the relative temperature which may be used. As a result, higher relative temperatures would tend to make the reaction conditions more stringent, while lower temperature less so. For additional details and explanation of stringency of hybridization reactions, see Ausubel, F. M. (1995), Current Protocols in Molecular Biology. John Wiley & Sons, Inc. Moreover, the person skilled in the art may follow the instructions given in the manual Boehringer Mannheim GmbH (1993) The DIG System Users Guide for Filter Hybridization, Boehringer Mannheim GmbH, Mannheim, Germany and in Liebl, W., Ehrmann, M., Ludwig, W., and Schleifer, K. H. (1991) International Journal of Systematic Bacteriology 41: 255-260 on how to identify DNA sequences by means of hybridization. Stringent conditions may be applied for any hybridization, i.e. hybridization occurs only if the probe is 70 % or more identical to the target sequence. Probes having a lower degree of identity with respect to the target sequence may hybridize, but such hybrids are unstable and will be removed in a washing step under stringent conditions, for example lowering the concentration of salt to 2 x SSC or, optionally and subsequently, to 0,5 x SSC, while the temperature is, in order of increasing preference, approximately 50°C - 68°C, approximately 52°C - 68°C, approximately 54°C - 68°C, approximately 56°C - 68°C, approximately 58°C - 68°C, approximately 60°C - 68°C, approximately 62°C - 68°C, approximately 64°C -68°C, approximately 66°C - 68°C. In an aspect, the temperature is approximately 64°C - 68°C or approximately 66°C - 68°C. It is possible to adjust the concentration of salt to 0.2 x SSC or even 0.1 x SSC. Nucleic acid sequences having a degree of identity with respect to the reference or wild type sequence of at least 70, 80, 90, 91, 92, 93, 94, 95, 96, 97, 98, 99 % may be isolated. In an aspect, the term variant of a nucleic acid sequence, as used herein, refers to any nucleic acid sequence that encodes the same amino acid sequence and variants thereof as the reference nucleic acid sequence, in line with the degeneracy of the genetic code.

The variant of the polypeptide has the ability to bind specifically to the ITPR1 autoantibodies found in patients and has at least 98 percent sequence identity with ITPR1 as defined by the Uniprot data base code Q14643.

The polypeptide, which comprises ITPR1 or a variant thereof, when used to carry out the teachings of the present invention, may be provided in any form and at any degree of purification, from tissues or cells comprising said polypeptide in an endogenous form, more preferably cells overexpressing the polypeptide, crude or enriched lysates of such cells, to purified and/or isolated polypeptide which is essentially pure. In a preferred embodiment, the polypeptide is a native polypeptide, wherein the term "native polypeptide", as used herein, refers to a folded polypeptide, more preferably to a folded polypeptide purified from tissues or cells, more preferably from mammalian cells or tissues, optionally from non-recombinant tissues or cells. The polypeptide may be a recombinant protein, wherein the term "recombinant", as used herein, refers to a polypeptide produced using genetic engineering approaches at any stage of the production process, for example by fusing a nucleic acid encoding the polypeptide to a strong promoter for overexpression in cells or tissues or by engineering the sequence of the polypeptide itself. The person skilled in the art is familiar with methods for engineering nucleic acids and polypeptides encoded (for example, described in Sambrook, J., Fritsch, E. F. and Maniatis, T. (1989), Molecular Cloning, CSH or in Brown T. A. (1986), Gene Cloning - an introduction, Chapman & Hall) and for producing and purifying native or recombinant polypeptides (for example Handbooks "Strategies for Protein Purification", "Antibody Purification", "Purifying Challenging Proteins", "Recombinant Protein Purification", "Affinity Chromatography", "Ion Exchange Chromatography", "Gel Filtration (Size Exclusion Chromatography)", "Hydrophobic Interaction Chromatography", "Multimodal Chromatography" (2009/2010), published by GE Healthcare Life Sciences, and in Burgess, R. R., Deutscher, M. P. (2009), Guide to Protein Purification). In a preferred embodiment, a polypeptide is pure if at least 60, 70, 80, 90, 95 or 99 percent of the polypeptide in the respective sample consists of said polypeptide as judged by SDS polyacrylamide gel electrophoresis followed by Coomassie blue staining and visual inspection.
If the polypeptide comprising ITPR1 or a variant thereof is provided in the form of tissue, it is preferred that the tissue is mammalian tissue, for example human, rat, primate, donkey, mouse, goat, horse, sheep, pig or cow, more preferably brain tissue. If a cell lysate is used, it is preferred that the cell lysate comprises the membranes associated with the surface of the cell. If said polypeptide is provided in the form of a recombinant cell, it is preferred that the recombinant cell is a eukaryotic cell such as a yeast cell, more preferably a cell from a multicellular eukaryote such as a plant, mammal, frog or insect, most preferably from a mammal, for example rat, human, primate, donkey, mouse, goat, horse, sheep, pig or cow. For example, the cell may be a HEK293 cell transfected with a nucleic acid functionally encoding the polypeptide. The person skilled in the art is familiar with methods for preparing, transfecting and culturing such cells, for example those described in Phelan, M. C. (2001), Basic Techniques in Mammalian Cell Tissue Culture, John Wiley.

The polypeptide comprises epitopes recognized by and/or binds specifically to autoantibodies binding to ITPR1. The person skilled in the art is familiar with guidelines used to design peptides having sufficient immunogenicity, for example those described in Jackson, D. C., Fitzmaurice, C. J., Brown, L. E., Zeng, W. (1999), Preparation and properties of totally synthetic immunogenes, Vaccine Volume 18, Issues 3-4, September 1999, Pages 355-361; and Black, M., Trent, A., Tirrell, M. and Olive, C. (2010), Advances in the design and delivery of peptide subunit vaccines with a focus on Toll-like receptor agonists, Expert Rev Vaccines, 2010 February; 9(2): 157-173. Briefly, it is desirable that the peptide meets as many as possible of the following requirements: (a) it has a high degree of hydrophilicity, (b) it comprises one or more residues selected from the group comprising aspartate, proline, tyrosine and phenylalanine, (c) is has, for higher specificity, no or little homology with other known peptides or polypeptides, (d) it needs to be sufficiently soluble and (e) it comprises no glycosylation or phosphorylation sites unless required for specific reasons. Alternatively, bioinformatics approaches may be followed, for example those described by Moreau, V., Fleury, C., Piquer, D., Nguyen, C., Novali, N., Villard, S., Laune, D., Granier, C. and Molina, F. (2008), PEPOP: Computational design of immunogenic peptides, BMC Bioinformatics 2008, 9:71.

The polypeptide, which comprises ITPR1 or a variant thereof, when used according to the present disclosure, may be provided in any kind of conformation. For example, the polypeptide may be an essentially unfolded, a partially or a fully folded polypeptide. In a preferred aspect, the polypeptide is folded in the sense that the epitopes that are essential for the binding to the autoantibody, or the protein or variant thereof in its entirety, adopt the fold adopted by the native protein in its natural environment. The person skilled in the art is familiar with methods suitable to determine whether or not a polypeptide is folded and if it is, which structure it has, for example limited proteolysis, NMR spectroscopy, CD spectroscopy or X-ray crystallography (see for example Banaszak L. J. (2008), Foundations of Structural Biology, Academics Press, or Teng Q. (2013), Structural Biology: Practical Applications, Springer), preferably multidimensional NMR spectroscopy is used.

The polypeptide may be a fusion protein which comprises amino acid sequences other than those taken from ITPR1, in particular a C-terminal or N-terminal tag, preferably a C-terminal tag, which is, as used herein, an additional sequence motif or polypeptide having a function that has some biological or physical function and may, for example, be used to purify, immobilize, precipitate or identify the polypeptide. The tag can be a sequence or domain capable of binding specifically to a ligand, for example a tag selected from the group comprising His tags, thioredoxin, maltose binding protein, glutathione-S-transferase, a fluorescence tag, for example from the group comprising green fluorescent protein.

The polypeptide may be an immobilized polypeptide. The term "immobilized", as used herein, refers to a molecule bound to a solid carrier insoluble in an aqueous solution, more preferably via a covalent bond, electrostatic interactions, encapsulation or entrapment, for example by denaturing a globular polypeptide in a gel, or via hydrophobic interactions, most preferably via one or more covalent bonds. Various suitable carriers, for example paper, polystyrene, metal, silicon or glass surfaces, microfluidic channels, membranes, beads such as magnetic beads, column chromatography media, biochips, polyacrylamide gels and the like have been described in the literature, for example in Kim, D., and Herr, A. E. (2013), Protein immobilizsation techniques for microfluidic assays, Biomicrofluidics 7(4), 041501. This way, the immobilized molecule, together with the insoluble carrier, may be separated from an aqueous solution in a straightforward manner, for example by filtration, centrifugation or decanting. An immobilized molecule may be immobilized in a reversible or irreversible manner. For example, the immobilization is reversible if the molecule interacts with the carrier via ionic interactions that can be masked by addition of a high concentration of salt or if the molecule is bound via a cleavable covalent bond such as a disulphide bridge which may be cleaved by addition of thiol-containing reagents. By contrast, the immobilization is irreversible if the molecule is tethered to the carrier via a covalent bond that cannot be cleaved in aqueous solution, for example a bond formed by reaction of an epoxide group and an amine group as frequently used to couple lysine side chains to affinity columns. The protein may be indirectly immobilized, for example by immobilizing an antibody or other entity having affinity to the molecule, followed by formation of a complex to the effect that the molecule-antibody complex is immobilized. Various ways to immobilize molecules are described in the literature, for example in Kim, D., Herr, and A. E. (2013), Protein immobilizsation techniques for microfluidic assays, Biomicrofluidics 7(4), 041501. In addition, various reagents and kits for immobilization reactions are commercially available, for example from Pierce Biotechnology.

It is essential that the sample used for the diagnosis in line with the present invention comprises antibodies, also referred to as immunglobulins. Typically the sample of a bodily fluid comprises a representative set of the entirety of the subject's immunglobulins. However, the sample, once provided, may be subjected to further processing which may include fractionation, centrifugation, enriching or isolating the entirety of immunglobulins or any immunglobulin class of the subject, which may affect the relative distribution of immunglobulins of the various classes.

The disease is paraneoplastic neurological syndrome, for example associated with small-cell lung cancer, lung cancer, ovarian cancer, breast carcinoma, Hodgkin's lymphoma, neuroblastoma, teratoma, non-Hodgkin lymphoma and bladder cancers.

The term "diagnosis", as used herein, refers to any kind of procedure aiming at obtaining information instrumental in the assessment whether a patient suffers or is likely or more likely than the average or a comparative subject, the latter preferably having similar symptoms, to suffer from certain a disease or disorder in the past, at the time of the diagnosis or in the future, to find out how the disease is progressing or is likely to progress in the future or to evaluate the responsiveness of a patient with regard to a certain treatment, for example the administration of immunosuppressive drugs. In other words, the term "diagnosis" comprises not only diagnosing, but also prognosticating and/or monitoring the course of a disease or disorder.

In many cases the mere detection, in other words determining whether or not detectable levels of the antibody are present in the sample, is sufficient for the diagnosis. If the autoantibody can be detected, this will be information instrumental for the clinician's diagnosis and indicates an increased likelihood that the patient suffers from a disease. In a preferred aspect, the relative concentration of the antibody in the serum, compared to the level that may be found in the average healthy subject, may be determined. While in many cases it may be sufficient to determine whether or not autoantibodies are present or detectable in the sample, the method carried out to obtain information instrumental for the diagnosis may involve determining whether the concentration is at least 0.1, preferably 0.2, 0.5, 1, 2, 5, 10, 20, 25, 50, 100, 200, 500, 1000, 10000 or 100000 times higher than the concentration found in the average healthy subject.

The person skilled in the art will appreciate that a clinician does usually not conclude whether or not the patient suffers or is likely to suffer from a disease, condition or disorders solely on the basis of a single diagnostic parameter, but needs to take into account other aspects, for example the presence of other autoantibodies, markers, blood parameters, clinical assessment of the patient's symptoms or the results of medical imaging or other non-invasive methods such as polysomnography, to arrive at a conclusive diagnosis. See Baenkler H. W. (2012), General aspects of autoimmune diagnostics, in Renz, H., Autoimmune diagnostics, 2012, de Gruyter, page 3. The value of a diagnostic agent or method may also reside the possibility to rule out one disease, thus allowing for the indirect diagnosis of another. In a preferred aspect, the meaning of any symptoms or diseases referred to throughout this application is in line with the person skilled in the art's understanding as of October 31th, 2014 as evidenced by text books and scientific publications.

Therefore, the term "diagnosis" does preferably not imply that the diagnostic methods or agents will be definitive and sufficient to finalize the diagnosis on the basis of a single test, let alone parameter, but may refer to a contribution to what is referred to as a "differential diagnosis", i. e. a systematic diagnostic procedure considering the likelihood of a range of possible conditions on the basis of a range of diagnostic parameters. Consequently, the method, polypeptide or use, optionally for determining whether a patient suffers from a disease, may comprise determining whether an autoantibody binding to ITPR1 is present in said sample, wherein said determining is performed by contacting the sample with the polypeptide and detecting whether binding occurs between said polypeptide and said autoantibody, preferably using a labeled secondary antibody, more preferably using a method from the group comprising radioimmunoassay, Western blot, line blot, ELISA, indirect and immunofluorescence, wherein said autoantibody binds to said polypeptide if present in the sample, and diagnosing the patient as suffering or being more likely to suffer from said neurological disorder or cancer if the autoantibody was determined to be present in the sample.

The term "diagnosis" may also refer to a method used to distinguish between two or more conditions associated with similar or identical symptoms.

The term "diagnosis" may also refer to a method used to choose the most promising treatment regime for a patient. In other words, the method or agent may relate to selecting a treatment regimen for a subject. For example, the detection of autoantibodies may indicate that an immunosuppressive therapy is to be selected, which may include administrating to the patient one or more immunosuppressive drugs.

A liquid sample comprising antibodies from a subject may be used to practice the method. Such a liquid sample may be any bodily fluid comprising a representative set of antibodies from the subject, preferably a sample comprising antibodies of the IgG immunglobulin class from the subject, more preferably from the group comprising IgG1, IgG2 and IgG4 subclasses, most preferably from the IgG4 subclass. For example, a sample may be cerebrospinal fluid (CSF), blood or blood serum, lymph, insterstitial fluid and is preferably serum or CSF, more preferably serum.

The step contacting a liquid sample comprising antibodies with the polypeptide may be carried out by incubating an immobilized form of said polypeptide in the presence of the sample comprising antibodies under conditions that are compatible with the formation of the complex comprising said polypeptide and an antibody, preferably an autoantibody, binding to the polypeptide. The liquid sample, then depleted of antibodies binding to the polypeptide may be removed subsequently, followed by one or more washing steps. Finally the complex comprising the antibody and the polypeptide may be detected. The term "conditions compatible with the formation of the complex" are conditions that allow for the specific antigen-antibody interactions to build up the complex comprising the polypeptide and the antibody. In a preferred aspect such conditions may comprise incubating the polypeptide in sample diluted 1:100 in PBS buffer for 30 minutes at 25 °C. The term "autoantibody", as used herein, refers to an antibody binding specifically to an endogenous molecule of the animal, preferably mammal, which produces said autoantibody, wherein the level of such antibody is more preferably elevated compared the average of any other antibodies binding specifically to such an endogenous molecule. The autoantibody is an autoantibody binding to ITPR1. Such an autoantibody may be isolated from samples taken from patients suffering from the neurological disorder characterized by two or more, preferably all symptoms selected from the group comprising mixed movement disorder, acute decline of visual performance, dysarthria and dysphagia.

In a preferred aspect, the detection of the complex for the prognosis, diagnosis, methods or test kit comprises the use of a method selected from the group comprising immunodiffusion techniques, immunoelectrophoretic techniques, light scattering immunoassays, light scattering immunoassays, agglutination techniques, labeled immunoassays such as those from the group comprising radiolabeled immunoassay, enzyme immunoassays, chemiluminscence immunoassays, and immunofluorescence techniques. The person skilled in the art is familiar with these methods, which are also described in the state of the art, for example in Zane, H. D. (2001), Immunology - Theoretical & Practical Concepts in Laboratory Medicine, W. B. Saunders Company, in particular in Chapter 14.

Alternatively, a sample comprising tissue comprising the polypeptide rather than a liquid sample may be used. The tissue sample is preferably from a tissue expressing endogenous ITPR1. Such a sample, which may be in the form of a tissue section fixed on a carrier, for example a glass slide for microscopic analysis, may then be contacted with the antibody, preferably autoantibody, binding to the polypeptide. The antibody is preferably labeled to allow for distinction from endogenous antibodies binding to the polypeptide, so that newly formed complexes may be detected and, optionally, quantified.

Any data demonstrating the presence or absence of the complex comprising the antibody and the polypeptide may be correlated with reference data. For example, detection of said complex indicates that the patient who provided the sample analyzed has suffered, is suffering or is likely to suffer in the future from a disease. If a patient has been previously diagnosed and the method for obtaining diagnostically relevant information is run again, the amount of complex detected in both runs may be correlated to find out about the progression of the disease and/or the success of a treatment. For example, if the amount of complex is found to increase, this suggests that the disorder is progressing, likely to manifest in the future and/or that any treatment attempted is unsuccessful.

A microplate, membrane ELISA, dot blot, or line blot may be used to carry out the diagnostic method according to the invention. The person skilled in the art is familiar with the experimental setup, which is described in the state of the art (Raoult, D., and Dasch, G. A. (1989), The line blot: an immunoassay for monoclonal and other antibodies. Its application to the serotyping of gram-negative bacteria. J. Immunol. Methods, 125 (1- 2), 57-65; WO2013041540). Briefly, the one or more antigen of interest, in the case of the present invention the polypeptide, may be attached to a carrier, for example nitrocellulose membrane, often in combination with additional antigens and controls. The nitrocellulose carrier is subsequently exposed to a sample comprising antibodies such as diluted serum. If the sample comprises an antibody binding to the antigen, a complex is formed which may be detected, preferably by incubation with a secondary antibody binding to the constant region of the first antibody, which secondary antibody comprises a detectable label, for example a radioactive isotope, a fluorescent dye or, in a preferred embodiment, an active enzyme fused or linked to the secondary antibody, such as alkaline phosphatase, which may be readily assayed using chromogenic substrates followed by simple visual examination. Suitable reagents, devices and software packages are commercially available, for example from EUROIMMUN, Lübeck, Germany.

Diagnosis or methods can contemplate the use of indirect immunofluorescence. The person skilled in the art is familiar with such techniques and the preparation of suitable samples, which are described in the state of the art (US4647543; Voigt, J., Krause, C., Rohwäder, E, Saschenbrecker, S., Hahn, M., Danckwardt, M., Feirer, C., Ens, K, Fechner, K, Barth, E, Martinetz, T., and Stöcker, W. (2012), Automated Indirect Immunofluorescence Evaluation of Antinuclear Autoantibodies on HEp-2 Cells," Clinical and Developmental Immunology, vol. 2012, doi:10.1155/2012/65105; Bonilla, E., Francis, L., Allam, F., et al., Immuno-fluorescence microscopy is superior to fluorescent beads for detection of antinuclear antibody reactivity in systemic lupus erythematosus patients, Clinical Immunology, vol. 124, no. 1, pp. 18-21, 2007). Briefly, a carrier, such as a cover glass for use in microscopy, is coated with cells or tissue sections comprising the antigen, the polypeptide may comprise one or more sequences of ITPR1 or a variant thereof. The carrier comprising the antigen is exposed to a patient sample comprising antibodies such as diluted serum. If the sample comprises an antibody binding to the antigen, the resulting complex may be detected, preferably by incubation with a secondary antibody comprising a fluorescent dye such as fluorescein, followed by visual examination using fluorescence microscopy. Suitable reagents, devices and software packages are commercially available, for example from EUROIMMUN, Lübeck, Germany.

A sample subjected to a test determining only whether an autoantibody binding to ITPR1 is present, but it is preferred that diagnostic methods, tests, devices and the like contemplate determining the presence of autoantibodies against a variety of antigens relating to neurological autoimmune disease or variants thereof, for example Hu, Yo, Ri, CV2, PNMA1, PNMA2, DNER/Tr, ARHGAP26, ITPR1, CARPVIII, Zic4, Sox1, MAG, MP0, MBP, GAD65, amphiphysin, recoverin, GABA A receptor, GABA B receptor, glycine receptor, gephyrin, IgLON5, DPPX, aquaporin-4, MOG, NMDA receptor, AMPA receptors, GRM1, GRM5, LGI1 and CASPR2. Therefore, the method, use, kit, device or the like may comprise two or more, preferably three, four, five or more antigens or variants thereof selected from the group comprising Hu, Yo, Ri, CV2, PNMA1, PNMA2, DNER/Tr, ARHGAP26, ITPR1, CARPVIII, Zic4, Sox1, MAG, MP0, MBP, GAD65, amphiphysin, recoverin, Homer 3, GABA A receptor, GABA B receptor, glycine receptor, gephyrin, IgLON5, DPPX, aquaporin-4, MOG, NMDA receptor, AMPA receptors, GRM1, CDR2L, GRM5, LGI1, CARPVIII and CASPR2, preferably from the group comprising Yo, CDR2L, DNER/Tr, ARHGAP26, Homer 3 and CARPVIII, more preferably ARHGAP26, Homer 3 and CARPVIII, which antigens are preferably immobilized, for example on a line blot.

According to the teachings of the present disclosure, an antibody, preferably an autoantibody binding to the polypeptide used for the diagnosis of a disease is provided. The person skilled in the art is familiar with methods for purifying antibodies, for example those described in Hermanson, G. T., Mallia, A. K., and Smith, P. K. (1992), Immobilized Affinity Ligand Techniques, San Diego: Academic Press. Briefly, an antigen binding specifically to the antibody of interest, which antigen is the polypeptide, is immobilized and used to purify, via affinity chromatography, the antibody of interest from an adequate source. A liquid sample comprising antibodies from a patient suffering from the neurological disorder identified by the inventors may be used as the source.

The term "antibody", as used herein, refers to any immuglobulin-based binding moieties, more preferably one comprising at least one immunoglobulin heavy chain and one immunoglobulin light chain,
including, but not limited to monoclonal and polyclonal antibodies as well as variants of an antibody, in particular fragments, which binding moieties are capable of binding to the respective antigen, more preferably binding specifically to it. The term "binding specifically", as used herein, means that the binding is stronger than a binding reaction characterized by a dissociation constant of 1 x 10⁻⁵ M, more preferably 1 x 10⁻⁷ M, more preferably 1 x 10⁻⁸ M, more preferably 1 x 10⁻⁹ M, more preferably 1 x 10⁻¹⁰ M, more preferably 1 x 10⁻¹¹ M, more preferably 1 x 10⁻¹² M, as determined by surface plasmon resonance using Biacore equipment at 25 °C in PBS buffer at pH 7. The antibody may be isolated or in a mixture comprising further antibodies, polypeptides, metabolites, cells and the like. In case the antibody is an autoantibody, it may be part of an autoantibody preparation which is heterogeneous or may be a homogenous autoantibody, wherein a heterogeneous preparation comprises a plurality of different autoantibody species as obtainable by preparation from the sera of human donors, for example by affinity chromatography using the immobilized antigen to purify any autoantibody capable of binding to said antigen. Preferably the antibody is an autoantibody, more preferably an autoantibody from the IgG class, most preferably from the group of subclasses comprising IgG1, IgG2 and IgG4, in particular IgG2. In another aspect, the antibody is a mammalian antibody, more preferably a primate antibody, most preferably a human antibody. The antibody may be glycosylated or non-glycosylated. The person skilled in the art is familiar with methods that may be used for the identification, production and purification of antibodies and variants thereof, for examples those described in EP 2 423 226 A2 and references therein. The antibody may be used according to the claims as a diagnostic agent, by itself, or in combination, for example in complex with the polypeptide.

A medical or diagnostic device comprising the (auto)antibody and/or the polypeptide may be used for diagnosis. Preferably such a medical or diagnostic device comprises the polypeptide in a form that allows contacting it with an aqueous solution, more preferably the liquid human sample, in a straightforward manner. In particular, the polypeptide may be immobilized on the surface of a carrier, which carrier comprises, but is not limited to glass plates or slides, biochips, microtiter plates, beads, for example magnetic beads, chromatography columns, membranes or the like. Exemplary medical devices include line blots, microplates and biochips. In addition to the polypeptide, the medical or diagnostic device may comprise additional polypeptides, for example positive or negative controls or known other antigens binding to autoantibodies of diagnostic value, particularly those related other diseases associated with one or more identical or similar symptoms.

The polypeptide data base code Q14643 may be used in the form of a cell comprising and/or expressing a nucleic acid encoding said polypeptide. If a nucleic acid comprising a sequence that encodes for the polypeptide or variant thereof is used, such a nucleic acid may be an unmodified nucleic acid. In a preferred aspect, the nucleic acid is a nucleic acid that, as such, does not occur in nature and comprises, compared to natural nucleic acid, at least one modification, for example an isotopic content or chemical modifications, for example a methylation, sequence modification, label or the like indicative of synthetic origin. In a preferred aspect, the nucleic acid is a recombinant nucleic acid or part or a nucleic acid, and is, in a more preferred embodiment, part of a vector, in which it may be functionally linked with a promoter that allows for expression, preferably overexpression of the nucleic acid.

In a preferred aspect, said nucleic acid is inside a cell capable of expressing it to the effect that the polypeptide or a variant thereof is made and, more preferably, routed to the surface of the cell. Said cell comprising the nucleic acid encoding the polypeptide may be used according to the present invention. The cell may be any kind of cell capable of expressing the nucleic acid, for example a prokaryotic or eukaryotic cell. In preferred aspect, the cell is a eukaryotic cell such as a yeast cell, a eukaryotic cell from a multicellular organism, for example an insect cell, more preferably a mammalian cell, for example a mouse cell, and most preferably a human cell.

The person skilled in the art is familiar with methods used to synthesize, modify and amplify such a nucleic acid and to transfect cells using such a nucleic acid, preferably in a vector that allows for the transient or permanent maintenance or expression of the nucleic acid in the cell. The person skilled in the art is also familiar with a variety of suitable vectors, of which are commercially available, for example from Origene. For example, a vector encoding for fusion constructs with a C-terminal GFP may be used. The cell may be of eukaryotic or prokaryotic origin and is preferably a mammalian cell, for example a HEK293, CHO or COS-7 cell. The cell comprising the nucleic acid encoding for the polypeptide may be a recombinant cell or an isolated cell wherein the term "isolated" means that the cell is enriched such that, compared to the environment of the wild type of said cell, fewer cells of other differentiation or species or in fact no such other cells are present.

The present invention is further illustrated by the following figures and non-limiting sequences and examples..
**Fig. 1** shows the result of an Indirect immunofluorescence analysis (IF A) of a positive patient serum that contains ITPR1 autoantibodies with the characteristic staining pattern indicated of the autoantibody.
**Fig. 2A****,** upper panel, shows the SDS-PAGE analysis of histo-immunoprecipitates from rat cerebellum. Three immunoprecipitated PCA positive reference sera showed, in addition to the immunoglobulins, a protein band corresponding to a molecular mass of approximately 300 kDa in SDS-PAGE stained with colloidal coomassie. The band was absent in the control samples.

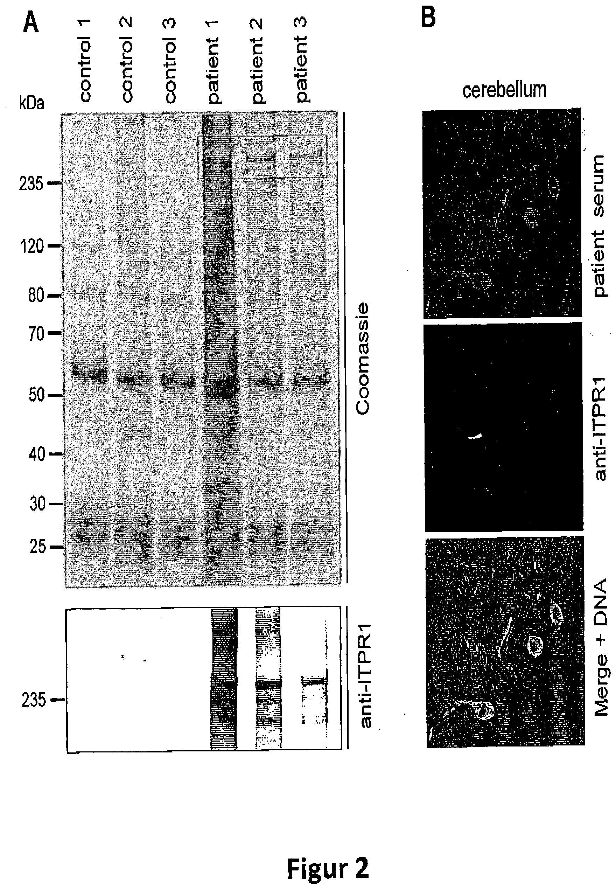

**Fig. 2A****,** bottom panel, shows a Western blot analysis with a polyclonal anti-ITPR1 antibody using the same samples. No band was visible if fractions from negative control sera were analysed.
**Fig. 2B** shows the results of double IF A staining using the polyclonal anti-ITPR1 antibody in addition to the index sera.

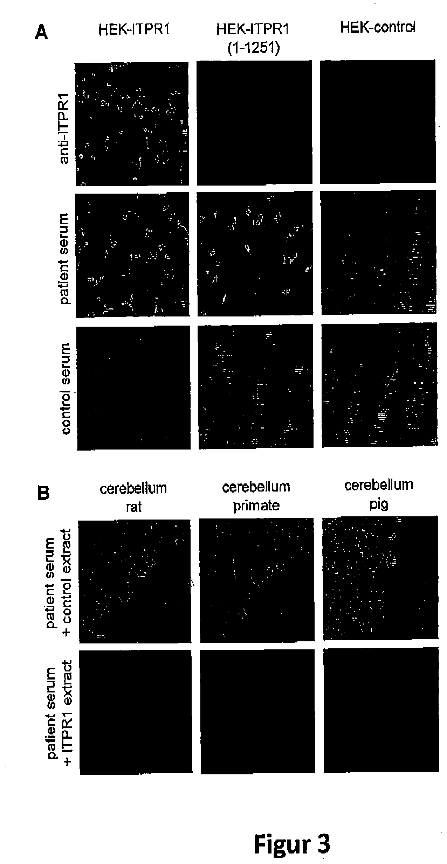

**Fig. 3A** shows analysis of patients' sera and controls by IF A with HEK293 expressing ITPR1 and with mock-transfected HEK293.
Fig. **3B** shows the competitive abolition of antibody binding to Purkinje cells by HEK293 fractions containing ITPR1. The upper row shows the IF A staining in the absence and the lower row shows the IFA staining in the presence of ITPR1.

### Example: Identification of ITPR1 autoantibodies in sera from patients suffering from neurological disease

The following example demonstrates that ITPR1 autoantibody is associated with neurological disease. It could be detected in three out of 30 patients having various symptoms associated with neurological disease, whilst it was absent in sera from 50 healthy subjects.

### 1. Materials & Methods

Reagents were obtained from Merck, Darmstadt, Germany or Sigma-Aldrich, Heidelberg, Germany if not specified otherwise.

### Indirect immunofluorescence assays

IFA was conducted using slides with a biochip array of >60 tissue and cell substrates including brain tissue cryosections (monkey, rat, and pig cerebellum, rat hippocampus, mouse whole-brain) and 28 recombinant HEK293 cells separately expressing established brain autoantigens including Yo/CDR2, CDR2L, DNER, ARHGAP26, CARP, Homer 3, Zic4, Sox1, GRM1, and GRM5 following the manufacturer's manual (Euroimmun, Lübeck, Germany). In some experiments, polyclonal antibody against ITPR1 (Dianova, Hamburg, Germany) was incubated in the first step followed by incubation with anti-rabbit IgG-Cy3 (Jackson Research, Suffolk, United Kingdom). Results were evaluated by two independent observers using a laser scanning microscope (LSM700, Zeiss, Jena, Germany). In competitive inhibition experiments, recombinant antigens were mixed with diluted serum sample 30 minutes prior to the IF A as described in Stöcker W, Otte M, Ulrich S, Normann D, Finkbeiner H, Stöcker K, et al. Autoimmunity to pancreatic juice in Crohn's disease. Results of an autoantibody screening in patients with chronic inflammatory bowel disease. Scand J Gastroenterol Suppl 1987;139:41-52.

### Histo-immunoprecipitation (Histo-IP)

Cerebellum from rat was dissected and shock-frozen in -160°C isopentane. The tissue was then cryosected (4 µm) with a SM2000R microtome (Leica Microsystems, Nussloch, Germany), placed on the entire surface of glass slides, and dried. Whole slides were then incubated with patient's serum (diluted 1:100) at 4°C for 3 hours followed by 3 washing steps with PBS containing 0.2% (w/v) Tween 20. Immunocomplexes were extracted from the sections by incubation in solubilization buffer (100 mmol/L tris-HCl pH 7.4, 150 mmol/L sodium chloride, 2.5 mmol/L EDTA, 0.5% (w/v) deoxycholate, 1% (w/v) Triton X-100 containing protease inhibitors) at room temperature for 30 minutes. Detached material was homogenized and centrifuged at 16,000 x g at 4°C for 15 minutes. The clear supernatants were then incubated with Protein G Dynabeads (ThermoFisher Scientific, Dreieich, Germany) at 4°C overnight to capture immunocomplexes. The beads were then washed 3 times with PBS, and eluted with PBS containing 5 mmol/L dithiothreitol and 1% (w/v) sodium dodecylsulfate at 95°C for 10 minutes, followed by SDS-PAGE and mass spectrometry or Western blot.

### SDS-PAGE and Western blot

Proteins were analyzed by SDS-PAGE using the NuPAGE system (ThermoFisher Scientific). Separated proteins were either identified by mass spectrometric analysis or electrotransferred onto a nitrocellulose membrane by tank blotting with transfer buffer (ThermoFisher Scientific) according to the manufacturer's instructions. The membranes were blocked with Universal Blot Buffer plus (Euroimmun) for 15 min and incubated with human serum or the polyclonal antibody against ITPR1 in Universal Blot Buffer plus for 3 hours, followed by 3 washing steps with Universal Blot Buffer (Euroimmun), a second incubation for 30 minutes with anti-rabbit IgG-AP (Sigma-Aldrich), 3 washing steps, and staining with NBT/BCIP substrate (Euroimmun).

### Mass spectrometry

Mass spectrometry sample preparation was performed as reported by Koy et al. (1). Unless otherwise indicated, hardware, software, MALDI targets, peptide standards and matrix reagents were obtained from Bruker Daltonics, Bremen, Germany.

Briefly, samples were reduced with dithiothreitol and carbamidomethylated with iodoacetamide prior to SDS-PAGE. Proteins were visualized with Coomassie Brilliant Blue G-250 and visible protein bands were excised and destained. After tryptic digest peptides were extracted and spotted with α-cyano-4-hydroxycinnamic acid onto a MTP AnchorChip™ 384 TF target.

MALDI-TOF/TOF measurements were performed with an Autoflex III smartbeam TOF/TOF200 System using flexControl 3.0 software. MS spectra for peptide mass fingerprinting (PMF) were recorded in positive ion reflector mode with 500 shots and in a mass range from 700 Da to 4000 Da. Spectra were calibrated externally with the commercially available Peptide Calibration Standard II, processed with flexAnalysis 3.0 and peak lists were analyzed with BioTools 3.2.

The Mascot search engine Mascot Server 2.3 (Matrix Science, London, UK) was used for protein identification by searching against the NCBI database limited to Mammalia. Search parameters were as follows: Mass tolerance was set to 80 ppm, one missed cleavage site was accepted, and carbamidomethylation of cysteine residues as well as oxidation of methionine residues were set as fixed and variable modifications, respectively. To evaluate the protein hits, a significance threshold of p<0.05 was chosen.

For further confirmation of the PMF hits two peptides of each identified protein were selected for MS/MS measurements using the WARP feedback mechanism of BioTools. Parent and fragment masses were recorded with 400 and 1000 shots, respectively. Spectra were processed and analyzed as described above with a fragment mass tolerance of 0.7 Da.

### Cloning and expression of ITPR1 in HEK293

The coding DNA for human ITPR1 (Genbank # BC172648, Source BioScience LifeSciences, Nottingham, UK) was transferred into the expression vector pTriEx-1 (Merck). The sequence of the resulting vector is referred to as SEQ ID NO 2. The receptor was expressed in the human cell line HEK293 after ExGen500-mediated transfection (ThermoFisher Scientific) according to the manufacturer's instructions. For the preparation of substrates for IF A, HEK293 were grown on sterile cover glasses, transfected, and allowed to express the recombinant proteins for 48 hours. Cover glasses were washed with PBS, fixed with acetone for 10 minutes at room temperature, air-dried, cut into millimeter-sized fragments (biochips) and used as substrates in IF A as above. Alternatively, cells were transfected in standard T-flasks and the cells were harvested after 5 days. The cell suspension was centrifuged at 1'500 x g, 4 °C for 20 minutes and the resulting sediment was extracted with Solubilization Buffer (see above). The extracted were stored in aliquots at -80 °C until further use.

### Results

### Characterization of a patients' sera

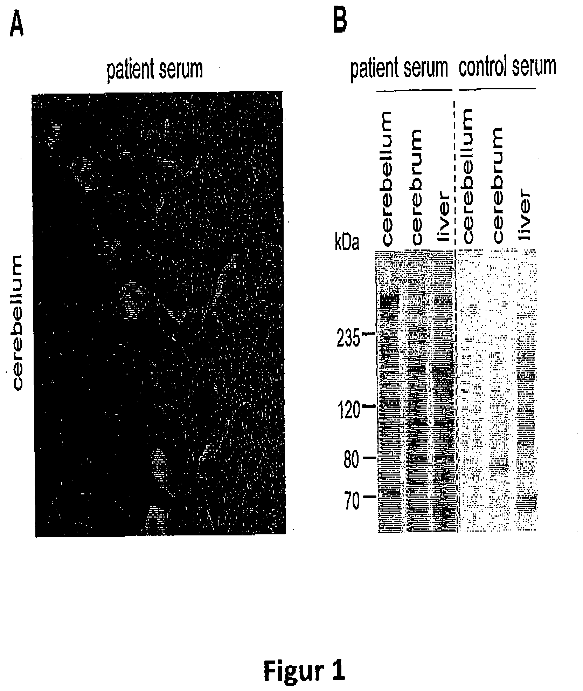

Indirect immunofluorescence analysis of a positive patient serum (henceforth referred to as index sera) revealed characteristic immunofluorescence patterns **(****Fig. 1****).**

### Identification of ITPR1 as the target autoantigen

In histo-immunoprecipitates from rat cerebellum three immunoprecipitated PCA positive reference sera showed, in addition to the immunoglobulins, a protein band corresponding to a molecular mass of approximately 300 kDa in SDS-PAGE stained with colloidal coomassie (**Fig. 2A****,** upper panel). The band was absent in the control samples. The precipitated protein was identified as ITPR1 by mass spectrometric analysis.

Western blot analysis with a polyclonal anti-ITPR1 antibody showed a strong reaction at 300 kDa of the immunoprecipitate obtained with the index sera, while there were no reactions with fractions obtained with control sera (**Fig. 2A**, bottom panel). When used for double staining in IFA the polyclonalclonal anti-ITPR1 antibody produced an overlay with the index sera **(****Fig. 2B****)**.

As a further proof for the correct antigen identification, the patients' sera and controls were then analyzed by IFA with HEK293 expressing ITPR1 and with mock-transfected HEK293 **(****Fig. 3A****).** All index sera but none of the controls reacted with the ITPR1-expressing cells similarly to the anti-ITPR1 antibody. In contrast, mock-transfection did not result in any antibody binding. The congruence of the patients' autoantibody target and ITPR1 was further demonstrated by the dose-dependent competitive abolition of antibody binding to Purkinje cells by HEK293 fractions containing ITPR1 **(****Fig. 3B****)** Antibody binding was unaffected when comparable fractions from mock-transfected HEK293 were used.

### Specificity of anti-ITPR1 autoantibodies

Sera from 30 patients with clinical symptoms indicating a neurological disease that were shown not contain a range of autoantibodies indicative of autoantibody-associated neurological diseases (anti-NMDAR, anti-Hu, anti-Yo, anti-Ri, anti-AQP4, anti-LGIl, anti-CASPR2) and from 50 healthy controls were analyzed by IFA. None of the sera produced a similar immunofluorescence pattern as the index sera on the recombinant substrate.

## Claims

1. An *in vitro* method for diagnosing a disease, preferably a disease associated with neurological symptoms, comprising the step detecting in a sample comprising antibodies from a patient an autoantibody binding to Inositol trisphosphate receptor type 1 (ITPR1) as defined by the Uniprot data base code Q14643,wherein the disease is paraneoplastic neurological syndrome preferably associated with breast carcinoma, wherein detection of the autoantibody indicates an increased likelihood that the patient suffers from the disease.

2. The method according to claim 1, wherein the sample is a bodily fluid comprising antibodies, preferably selected from the group comprising whole-blood, serum, cerebrospinal fluid.

3. Use of a polypeptide comprising amino acids 1-1251 of ITPR1 as defined by the Uniprot data base code Q14643 or a variant thereof having the ability to bind specifically to an autoantibody to ITPR1 as defined by the Uniprot data base code Q14643 for the *in vitro* diagnosis of a disease, comprising the step detecting autoantibodies binding to ITPR1 as defined by the Uniprot data base code Q14643, wherein the disease is paraneoplastic neurological syndrome, preferably associated with breast carcinoma, wherein the variant has at least 98 percent sequence identity with ITPR1 as defined by the Uniprot data base code Q14643.

4. Use of an autoantibody, preferably an isolated autoantibody, binding to polypeptide ITPR1 as defined by the Uniprot data base code Q14643, wherein the autoantibody is preferably in complex with said polypeptide, for the *in vitro* diagnosis of a disease, wherein the disease is paraneoplastic neurological syndrome, preferably associated with breast carcinoma, and wherein said autoantibody to ITPR1 is detected.

5. Use of a medical or diagnostic device comprising a polypeptide comprising amino acids 1-1251 of ITPR1 as defined by the Uniprot data base code Q14643 or a variant thereof having the ability to bind specifically to an autoantibody to ITPR1 as defined by the Uniprot data base code Q14643 for the *in vitro* diagnosis of a disease, wherein the disease is paraneoplastic neurological syndrome, preferably associated with breast carcinoma, and wherein an autoantibody to ITPR1 as defined by the Uniprot data base code Q14643 is detected, wherein the variant has at least 98 percent sequence identity with ITPR1 as defined by the Uniprot data base code Q14643.

6. The method or use according to any of claims 1 to 5, wherein the polypeptide is provided in the form of a cell comprising a nucleic acid encoding said polypeptide, wherein the nucleic acid is part of a vector, in which it is functionally linked with a promoter that allows for expression, or in the form of a tissue comprising said polypeptide.

7. The method or use according to any of claims 1 to 6, wherein the polypeptide is a recombinant and/or isolated polypeptide.

## Patentansprüche

1. Ein *in vitro* Verfahren zur Diagnose einer Krankheit, vorzugsweise einer Krankheit, die mit neurologischen Symptomen assoziiert ist, umfassend den Schritt des Nachweisens eines Autoantikörpers, der an den Inositol-Trisphosphat-Rezeptor Typ 1 (ITPR1), wie durch den Uniprot-Datenbankcode Q14643 definiert, bindet, in einer Probe, die Antikörper von einem Patienten enthält, wobei die Krankheit ein paraneoplastisches neurologisches Syndrom ist, das vorzugsweise mit einem Brustkarzinom assoziiert ist, wobei der Nachweis des Autoantikörpers eine erhöhte Wahrscheinlichkeit anzeigt, dass der Patient an der Krankheit leidet.

2. Das Verfahren nach Anspruch 1, wobei die Probe eine Körperflüssigkeit ist, die Antikörper umfasst, die vorzugsweise aus der Gruppe ausgewählt ist, die Vollblut, Serum, Cerebrospinalflüssigkeit umfasst.

3. Verwendung eines Polypeptids, umfassend die Aminosäuren 1-1251 von ITPR1, wie durch den Uniprot-Datenbankcode Q14643 definiert, oder eine Variante davon mit der Fähigkeit, spezifisch an einen Autoantikörper gegen ITPR1, wie durch den Uniprot-Datenbankcode Q14643 definiert, zu binden, für die *in vitro* Diagnose einer Krankheit, umfassend den Schritt des Nachweisens von Autoantikörpern, die an ITPR1, wie durch den Uniprot-Datenbankcode Q14643 definiert, binden, wobei die Krankheit ein paraneoplastisches neurologisches Syndrom ist, das vorzugsweise mit einem Brustkarzinom assoziiert ist, wobei die Variante mindestens 98 Prozent Sequenzidentität mit ITPR1, wie durch den Uniprot-Datenbankcode Q14643 definiert, aufweist.

4. Verwendung eines Autoantikörpers, vorzugsweise eines isolierten Autoantikörpers, der an das Polypeptid ITPR1, wie durch den Uniprot-Datenbankcode Q14643 definiert, bindet, wobei der Autoantikörper vorzugsweise in einem Komplex mit dem Polypeptid vorliegt, für die *in vitro* Diagnose einer Krankheit, wobei die Krankheit ein paraneoplastisches neurologisches Syndrom ist, das vorzugsweise mit einem Brustkarzinom assoziiert ist, und wobei der Autoantikörper gegen ITPR1 nachgewiesen wird.

5. Verwendung einer medizinischen oder diagnostischen Vorrichtung, die ein Polypeptid umfasst, das die Aminosäuren 1-1251 von ITPR1, wie durch den Uniprot-Datenbankcode Q14643 definiert, oder eine Variante davon mit der Fähigkeit, spezifisch an einen Autoantikörper gegen ITPR1, wie durch den Uniprot-Datenbankcode Q14643 definiert, zu binden, umfasst, für die *in vitro* Diagnose einer Krankheit, wobei die Erkrankung ein paraneoplastisches neurologisches Syndrom ist, das vorzugsweise mit einem Brustkarzinom assoziiert ist, und wobei ein Autoantikörper gegen ITPR1, wie durch den Uniprot-Datenbankcode Q14643 definiert, nachgewiesen wird, wobei die Variante mindestens 98 Prozent Sequenzidentität mit ITPR1, wie durch den Uniprot-Datenbankcode Q14643 definiert, aufweist.

6. Das Verfahren oder die Verwendung nach einem der Ansprüche 1 bis 5, wobei das Polypeptid in Form einer Zelle bereitgestellt wird, die eine für das Polypeptid kodierende Nukleinsäure umfasst, wobei die Nukleinsäure Teil eines Vektors ist, in dem sie funktionell mit einem Promotor verbunden ist, der die Expression ermöglicht, oder in Form eines Gewebes, das das Polypeptid umfasst.

7. Das Verfahren oder die Verwendung nach einem der Ansprüche 1 bis 6, wobei das Polypeptid ein rekombinantes und/oder isoliertes Polypeptid ist.

## Revendications

1. Procédé *in vitro* destiné à diagnostiquer une maladie, de préférence une maladie associée à des symptômes neurologiques, comprenant l'étape de détection dans un échantillon, qui comprend des anticorps provenant d'un patient, d'un auto-anticorps se liant au récepteur de l'inositol triphosphate de type 1 (ITPR1) tel que défini par le code Q14643 de la base de données Uniprot, dans lequel la maladie est un syndrome neurologique paranéoplasique associé de préférence à un carcinome du sein, dans lequel la détection de l'auto-anticorps indique une vraisemblance accrue que le patient soit atteint de la maladie.

2. Procédé selon la revendication 1, dans lequel l'échantillon est un liquide corporel comprenant des anticorps, de préférence choisi dans le groupe comprenant le sang entier, le sérum, le liquide céphalorachidien.

3. Utilisation d'un polypeptide comprenant les acides aminés 1 à 1251 de l'ITPR1, tel que défini par le code Q14643 de la base de données Uniprot, ou d'un variant de celui-ci ayant la faculté de se lier spécifiquement à un auto-anticorps contre l'ITPR1, tel que défini par le code Q14643 de la base de données Uniprot, pour le diagnostic *in vitro* d'une maladie, comprenant l'étape de détection d'auto-anticorps se liant à l'ITPR1 tel que défini par le code Q14643 de la base de données Uniprot, dans laquelle la maladie est un syndrome neurologique paranéoplasique, de préférence associé à un carcinome du sein, dans laquelle le variant a une identité de séquence d'au moins 98 pour cent avec l'ITPR1 tel que défini par le code Q14643 de la base de données Uniprot.

4. Utilisation d'un auto-anticorps, de préférence un auto-anticorps isolé, se liant à un ITPR1 polypeptidique, tel que défini par le code Q14643 de la base de données Uniprot, dans laquelle l'auto-anticorps est de préférence en complexe avec ledit polypeptide, pour le diagnostic *in vitro* d'une maladie, dans laquelle la maladie est un syndrome neurologique paranéoplasique, de préférence associé à un carcinome du sein, et dans laquelle est détecté ledit auto-anticorps contre l'ITPR1.

5. Utilisation d'un dispositif médical ou diagnostique comprenant un polypeptide, qui comprend les acides aminés 1 à 1251 de l'ITPR1 tel que défini par le code Q14643 de la base de données Uniprot, ou un variant de celui-ci ayant la faculté de se lier spécifiquement à un auto-anticorps contre l'ITPR1, tel que défini par le code Q14643 de la base de données Uniprot, pour le diagnostic *in vitro* d'une maladie, dans laquelle la maladie est un syndrome neurologique paranéoplasique, de préférence associé à un carcinome du sein, et dans laquelle est détecté un auto-anticorps contre l'ITPR1 tel que défini par le code Q14643 de la base de données Uniprot, dans laquelle le variant a une identité de séquence d'au moins 98 pour cent avec l'ITPR1 tel que défini par le code Q14643 de la base de données Uniprot.

6. Procédé ou utilisation selon l'une quelconque des revendications 1 à 5, dans lequel/laquelle le polypeptide est fourni sous la forme d'une cellule comprenant un acide nucléique qui code pour ledit polypeptide, dans lequel/laquelle l'acide nucléique fait partie d'un vecteur, dans lequel il est fonctionnellement lié à un promoteur qui permet d'obtenir une expression, ou est sous la forme d'un tissu comprenant ledit polypeptide.

7. Procédé ou utilisation selon l'une quelconque des revendications 1 à 6, dans lequel/laquelle le polypeptide est un polypeptide recombinant et/ou isolé.
